# EUROPÄISCHE PATENTSCHRIFT

(11) **EP 3 491 173 B1**
(45) Veröffentlichungstag und Bekanntmachung des Hinweises auf die Patenterteilung: **13.04.2022**
(21) Anmeldenummer: 17746057.3
(22) Anmeldetag: 26.07.2017
(51) Int. Cl.: C25B 1/04, C25B 15/08, C07C 1/02, C07C 29/152, C07C 31/04, C07C 29/78, C07C 29/80, C10L 3/08

(54) **VERFAHREN UND ANLAGE ZUR HERSTELLUNG VON METHANOL**
METHOD AND SYSTEM FOR THE PRODUCTION OF METHANOL
PROCÉDÉ ET DISPOSITIF DE FABRICATION DU MÉTHANOL

(30) Priorität: 26.07.2016 DE 102016213668
(43) Veröffentlichungstag der Anmeldung: 05.06.2019
(73) Patentinhaber: thyssenkrupp Industrial Solutions AG, 45143 Essen (DE); thyssenkrupp AG, 45143 Essen (DE)
(72) Erfinder: SCHULZ, Alexander, 60439 Frankfurt (DE); SCHIRRMEISTER, Steffen, 45472 Mülheim an der Ruhr (DE)
(74) Vertreter: thyssenkrupp Intellectual Property GmbH
(86) Internationale Anmeldenummer: PCT/EP2017/068859
(87) Internationale Veröffentlichungsnummer: WO 2018/019875

(56) Entgegenhaltungen:
- WO-A1-2013/029701
- WO-A1-2014/173452
- DE-U1-202010 012 734

## Beschreibung

Die vorliegende Erfindung betrifft ein Verfahren zur Herstellung von Methanol durch Umsetzung von Kohlendioxid und Wasserstoff sowie eine in diesem Verfahren verwendbare Anlage, bei dem ein bei der Methanolsynthesereaktion erhaltener Produktstrom einem Hochdruckabscheider und/oder einem Niederdruckabscheider zugeführt wird, in dem ein Gasstrom von einem Methanol-haltigen Produktstrom abgetrennt wird.

Im Rahmen der Energiewende von fossil erzeugter elektrischer Energie zu regenerativ erzeugter Energie fallen Stromspitzen an, die nicht mehr in konventionelle elektrische Netze integriert werden können. Verschiedene Technologien zur Speicherung bzw. anderweitigen Nutzung von Stromüberschüssen in Zeiten eines Überangebotes erneuerbarer Energien werden unter dem Begriff "Power-to-X" zusammengefasst. Eine Variante dieser Technologie wird mit dem Begriff "Power-to-Gas" bezeichnet. Dabei wird zunächst der überschüssige Strom aus regenerativen Energiequellen mit Hilfe einer Wasserelektrolyse in Wasserstoff umgewandelt. Anschließend wird der Wasserstoff beispielsweise mit Kohlendioxid zu Methan oder Methanol umgesetzt. Das erzeugte Methan kann zum Beispiel in das Erdgasnetz eingespeist werden. Alternativ dazu kann der elektrolytisch gewonnene Wasserstoff auch mit Stickstoff zu Ammoniak umgesetzt werden (diese Variante bezeichnet man auch als "Power-to-Ammonia"). Auf diese Weise kann die Energie chemisch in Form von Ammoniak gespeichert werden.

Im Vergleich zu den zum Teil recht großen Elektrolyseanlagen im Megawatt-Bereich sind diese Power-to-X Anlagen relativ klein [beispielsweise in der Größenordnung von einigen 1.000 Tonnen/Jahr bis zu mehreren 10.000 Tonnen/Jahr (t/a)]. Eine wirtschaftliche und umweltgerechte Entsorgung von Purgegas- und Abgasströmen solcher Anlagen, die weitestgehend autark betrieben werden, ist über konventionelle Fackelsysteme kaum gegeben. Als Purgegase bezeichnet man in einem Gasstrom enthaltene Gase, die sich bei einer Umsetzung von Eduktgasen zu einem Produktgas gegenüber der gewünschten Reaktion inert verhalten. Da bei derartigen Reaktionen zumeist die miteinander reagierenden Gase im Kreislauf geführt werden, müssen die inerten Gase und/oder unerwünschte Nebenprodukte gegebenenfalls als Purgegase aus dem Synthesekreislauf entfernt werden, damit sie sich nicht im Kreislauf anreichern.

Aus der DE 20 2010 012 734 U1 ist ein Verfahren bekannt, bei dem man mittels aus einer regenerativen Energiequelle erzeugter elektrischer Energie in einer Elektrolyseeinheit durch Elektrolyse von Wasser Wasserstoff erzeugt und diesen Wasserstoff anschließend in einer Reaktoreinheit katalytisch mit Kohlendioxid umsetzt, um so Methanol oder Methan herzustellen. Das dabei erhaltene Methan oder Methanol wird als kohlenwasserstoffhaltiger Energieträgerstrom in einer Brennkammer verbrannt und die thermische Energie des bei der Verbrennung gebildeten Rauchgases wird dann in einem Gasturbinenprozess oder einem Dampfturbinenprozess zur Erzeugung elektrischer Energie genutzt.

In der Druckschrift WO 2014/173452 A1 werden ein Verfahren und eine Reaktoranlage zur Synthese von Methanol mit Kreisgas- und Purgegasrückführung beschrieben, wobei das Methanol in einer exothermen Reaktion aus Kohlendioxid und Wasserstoff hergestellt wird und der dabei eingesetzte Wasserstoff durch Elektrolyse von Wasser gewonnen wird. Das Produktgasgemisch aus der Methanolsynthese wird hier am Produktauslass in einen Anteil methanolhaltiges Produkt, einen Kreisgasanteil und einen Purgegasanteil getrennt, wobei ein Teil des Purgegasanteils zu einer Eingangsstufe zurückgeführt und erneut durch den Reaktor geführt wird. Das Purgegas enthält Kohlendioxid, Wasserstoff und Kohlenmonoxid. Bei einer Variante des Verfahrens gibt es einen Purgestrom, der über eine Leitung in die Atmosphäre abgegeben wird. Eine katalytische Abgasreinigung ist hier nicht vorgesehen.

Aus der US 2007/0282021 A1 ist ein Verfahren zur Herstellung von Ethanol durch Umsetzung von Kohlendioxid mit Wasserstoff bekannt, bei dem neben Ethanol auch andere organische Verbindungen wie Methanol und höhere Alkohole als Nebenprodukte anfallen. Dieses bekannte Verfahren ist vergleichsweise unspezifisch, da das erwünschte Produkt Ethanol nur in einem Anteil von 52 % erhalten wird, während außerdem 26 % Methanol und weitere Alkohole mit bis zu sechs C-Atomen entstehen. Das Produktgemisch kann zur Aufbereitung zunächst gestrippt und dann destilliert werden, um dann ein gereinigtes Ethanol mit einem Reinheitsgrad von 87 % zu erhalten.

Die DE 20 2010 012 734 U1 beschreibt ein Verfahren zur katalytischen Erzeugung von Methanol oder Methan aus einem elektrolytisch erzeugten Wasserstoffstrom und Kohlendioxid. Dieses Verfahren dient dazu, Erzeugungsspitzen bei der Erzeugung von elektrischer Energie aus regenerativen Energiequellen auszugleichen. Das bei diesem Verfahren erzeugte Methanol wird anschließend in einer Brennkammer unter Zufuhr eines Sauerstoffstroms verbrannt, so dass es nicht darauf ankommt, ein Methanol mit einem hohen Reinheitsgrad zu produzieren, denn das Methanol gelangt nicht als Syntheseprodukt in den Handel. Die Aufbereitung des methanolhaltigen Produktstroms wird in dieser Druckschrift nicht näher beschrieben. Eine katalytische Abgasreinigung ist bei diesem bekannten Verfahren nicht vorgesehen.

Aufgabe der vorliegenden Erfindung ist es, ein Verfahren zur Herstellung von Methanol durch Umsetzung von Kohlendioxid und Wasserstoff hinsichtlich des Wirkungsgrads, des Energiebedarfs, der anfallenden Abgasströme und Abwasserströme und der Produktreinheit zu optimieren.

Die Lösung dieser Aufgabe liefert ein Verfahren zur Herstellung von Methanol durch Umsetzung von Kohlendioxid und Wasserstoff mit den Merkmalen des Anspruchs 1.

Gegenstand der vorliegenden Erfindung ist vorrangig ein Verfahren sowie eine Anlage zur Herstellung von Methanol. Grundsätzlich sind die hierin beschriebene Verfahrensweise sowie die Anlagenkonzeption aber auch für die Herstellung anderer Alkohole, insbesondere niederer Alkohole wie beispielsweise Ethanol geeignet.

Erfindungsgemäß ist vorgesehen, dass der nach dem Hochdruckabscheider und/oder nach dem Niederdruckabscheider erhaltene Methanol-haltige Produktstrom anschließend mindestens einem Destillationsschritt zugeführt wird, in dem mindestens eine Komponente, insbesondere Wasser von dem Methanol-haltigen Produktstrom abgetrennt wird.

Gemäß einer bevorzugten Weiterbildung des Verfahrens wird ein mindestens eine abgetrennte leichtflüchtige Komponente enthaltender Gasstrom als Abgas vollständig oder nur teilweise aus dem System abgeführt und/oder dieser Gasstrom oder ein Teil dieses abgetrennten Gasstroms wird wieder in die Methanolsynthesereaktion zurückgeführt.

Das erfindungsgemäße Verfahren kann gemäß einer von mehreren möglichen alternativen Varianten mit einer derartigen Anlagenkonfiguration so betrieben werden, dass ein Abgasstrom völlig vermieden oder zumindest minimiert ist. Die durch die optimierte Verschaltung anfallenden Abgasströme sind bei dieser Variante der erfindungsgemäßen Methanolherstellung so klein, dass im Normalbetrieb weder eine Fackel noch eine Abgasaufbereitung notwendig wird.

Bei einer möglichen Variante der Erfindung ist bevorzugt eine katalytische Abgasreinigung eines Abgas- und/oder Purgegasstroms vorgesehen.

Ist eine solche katalytische Abgasreinigung vorgesehen, dann umfasst diese vorzugsweise eine katalytische Nachverbrennung. Diese katalytische Nachverbrennung erfolgt mit Zufuhr eines Zusatzbrennstoffes.

Sofern eine katalytische Nachverbrennung des Abgasstroms vorgesehen ist, kann die Emission insbesondere von Kohlenwasserstoffen in die Umwelt reduziert werden. Das mit den Schadstoffen belastete Abgas wird dann durch eine Einrichtung geleitet, in der sich wenigstens ein Katalysator befindet. Die katalytische Nachverbrennung arbeitet nach dem Prinzip der heterogenen Katalyse. Vorteilhaft ist, dass es sich um ein Verfahren zur Abgasreinigung handelt, welches bei einer vergleichsweise niedrigen Reaktionstemperatur erfolgen kann. Die in dem Abgasstrom enthaltenen Kohlenwasserstoffe werden in der Regel zu Kohlendioxid und Wasser oxidiert. Diese Oxidation kann entweder direkt oder über Zwischenstufen erfolgen.

Als Zusatzbrennstoff bei der katalytischen Nachverbrennung kann beispielsweise Luft oder Sauerstoff, der bei der Wasserelektrolyse neben H₂ anfällt, eingesetzt werden. Durch den Zusatz von Sauerstoff werden Oxidationsprozesse unterstützt und/oder beschleunigt.

Bei den Varianten, bei denen kein oder nur ein minimaler Abgasstrom aus dem System abgeführt wird, können bei der Destillation oder Kondensation abgetrennte leichtflüchtige Komponenten wieder in die Methanolsynthesereaktion zurückgeführt werden. Dadurch werden eine Erhöhung der Produktausbeute und eine Verbesserung des Wirkungsgrads des Verfahrens insgesamt erzielt. Zudem führen diese Maßnahmen zur Vermeidung bzw. starken Verminderung von Umweltbelastungen.

Das im Destillationsschritt von dem Methanol- oder Methan-haltigen Produktstrom abgetrennte Wasser kann ebenfalls zurückgeführt werden, anstatt es als Abwasser aus dem System abzuführen, wobei dies insbesondere bei einer bevorzugten Verfahrensvariante interessant ist, bei der der bei der Synthese von Methanol als Edukt eingesetzte Wasserstoff zuvor durch Elektrolyse von Wasser gewonnen wurde. Bei dieser Variante des erfindungsgemäßen Verfahrens kann beispielsweise zuvor insbesondere aus regenerativen Energien gewonnener elektrischer Strom zur Elektrolyse von Wasser eingesetzt werden. Der dabei erzeugte Wasserstoff kann danach mit Kohlendioxid zu Methanol umgesetzt werden (Methanolsynthese). Das dabei entstehende Produkt wird anschließend durch geeignete Trennverfahren aufbereitet.

Prinzipiell kann bei den erfindungsgemäßen Verfahren das Prozesswasser direkt in eine Kanalisation gegeben werden. Bei dezentralen Anlagen kann allerdings eine Entsorgung aufwändig sein. Daher ist es vorteilhaft, wenn gemäß der zuvor genannten Verfahrensvariante das Prozesswasser im Prozess wieder verwertet werden kann und nicht entsorgt werden muss. Durch die Verwertung des Prozesswassers wird der Bedarf an Frischwasser signifikant reduziert, beispielsweise um mehr als 30 %.

Eine Aufbereitung des Prozesswassers kann bei der Variante mit Herstellung des Wasserstoffs durch Wasserelektrolyse in Abhängigkeit von der eingesetzten Elektrolysetechnologie notwendig sein. Diese Wasseraufbereitung kann beispielsweise eine Entfernung von im Wasser enthaltenen Methanol durch ein Membranverfahren umfassen. Aus dem extern zugeführten Frischwasser können Salze beispielsweise durch lonenaustausch und/oder Umkehrosmose entfernt werden.

Das bei der Methanolsynthesereaktion als Edukt eingesetzte Kohlendioxid kann beispielsweise mindestens zum Teil kryogen zur Verfügung stehen. Für die Verdampfung und Erwärmung des Kohlendioxids kann in diesem Fall die Wärme aus dem Rücklauf eines im Prozess verwendeten Kühlmediums genutzt werden. Diese Kopplung der Kühlung eines Kühlmediums aus einem geschlossenen Kühlkreislauf mit der Verdampfung und Erwärmung von CO₂ ist eine vorteilhafte Variante, da dann keine separate Wärmeabfuhr für die CO₂-Verdampfung notwendig ist und eine separate aufwändige Kühlung des Kühlmediums entfällt. Als Kühlmedium kann beispielsweise ein Glykol/Wasser-Gemisch verwendet werden. Dieses kann zum Beispiel in einer vorgeschalteten Luft- oder Wasserkühlung vorgekühlt werden.

Gemäß einer möglichen bevorzugten Variante des Verfahrens ist eine der Methanolsynthesereaktion vorgeschaltete Misch- und Verdichtersektion vorgesehen, welcher von extern Kohlenmonoxid oder Wasserstoff oder ein Kohlenstoffoxide und Wasserstoff haltiges Synthesegas zugeführt wird. Bei der Methanolsynthese wird durch die Zugabe von Kohlenmonoxid die Laufzeit des Katalysators erhöht, denn die Deaktivierung des Katalysators verläuft bei reinem Kohlendioxid schneller. Steht an einem Standort Wasserstoff zur Verfügung, kann dieser dem System zugeführt und somit die Wasserelektrolyse entlastet werden, so dass der Strombedarf gesenkt wird.

Durch Kombination des zuvor beschriebenen Systems beispielsweise mit einer konventionellen Synthesegaserzeugung, beispielsweise durch Steam Reforming, Combined Reforming oder katalytische POX (autothermes Reforming) kann eine weitere Optimierung des Verfahrens erfolgen. Die optimale Position für die Zugabe externer Gase hängt unter anderem von dem Druckniveau ab, bei dem das Gas anliegt.

Wenn durch die externe Gaszugabe bezüglich der Produktsynthese inerte Komponenten in das System gelangen, die in den nachfolgenden Trennprozessen nicht im Gemisch mit dem Produkt und nicht im Wasser anfallen, beispielsweise Stickstoff, kann ein kleiner Abgasstrom notwendig werden, um eine Akkumulation der Inert-Komponenten im System zu vermeiden. Bei reinen Komponenten ist hingegen kein oder ein minimaler Abgasstrom vorgesehen.

Bei einer Basisversion des erfindungsgemäßen Verfahrens kann man beispielsweise als Ausgangskomponenten kryogenes Kohlendioxid sowie elektrischen Strom vorsehen, mittels dessen man den für die Synthese notwendigen Wasserstoff elektrolytisch aus Wasser erzeugt. Es sind jedoch viele alternative Varianten im Rahmen der Erfindung möglich, wenn zum Beispiel in einer Kläranlage, einem Kraftwerk oder dergleichen CO₂ schon gasförmig vorliegt oder wenn zum Beispiel über eine Pipeline oder am Standort einer Chemiefabrik H₂ zur Verfügung steht. In diesen Fällen können die Verdampfung und Aufwärmung des CO₂ bzw. die Wiederverwertung des Prozesswassers im System entfallen.

Im Rahmen von Weiterbildungen des Verfahrens gemäß der vorliegenden Erfindung können neben der Destillation verschiedene weitere Trennprozesse zur Aufbereitung und Reinigung des Produktstroms vorgesehen sein. Beispielsweise kann ein bei der Methanolsynthesereaktion erhaltener Produktstrom zunächst einem Niederdruckabscheider zugeführt werden und ein in diesem Niederdruckabscheider von dem Methanol-haltigen Produktstrom abgetrennter Gasstrom kann anschließend dem Destillationsschritt zugeführt werden. Eine direkte Zugabe der Gasphase aus einem Niederdruckabscheider in eine Destillationsvorrichtung hat die Vorteile der Minimierung der Abgasmenge und der Erhöhung des Gesamtwirkungsgrads der Anlage.

Erfindungsgemäss werden in dem Destillationsschritt abgetrennte leichtflüchtige Komponenten teilweise oder vollständig in eine der Methanolsynthese vorgeschaltete Misch- und Verdichtersektion zurückgeführt.

Diese Misch- und Verdichtersektion kann eine oder mehrere hintereinandergeschaltete Verdichterstufen umfassen. Dies ist unter anderem abhängig von dem Reaktordruck. Viele Wasserelektrolysen arbeiten bei erhöhtem Druck von beispielsweise etwa 10 bara, so dass CO₂ und H₂ vor dem Verdichter gemischt werden können. In einem solchen Fall müsste ein recycelter Strom vor der Zugabe zu CO₂ und H₂ zunächst in einer ersten Verdichterstufe komprimiert werden.

Bei anderen Varianten der Wasserelektrolyse fällt hingegen der Wasserstoff nur bei geringem Überdruck an, beispielsweise im Bereich von einigen 100 mbar. In diesem Fall ist es vorteilhaft, zunächst den rückgeführten Strom mit H₂ zu mischen und dann in einer ersten Verdichterstufe zu komprimieren. Danach könnte dann CO₂ zugegeben und das Gemisch dann auf den gewünschten Druck verdichtet werden.

Prinzipiell kann ein Verdichter mit mehreren Stufen oder alternativ auch zwei oder mehrere getrennte Verdichter eingesetzt werden.

Bei der erfindungsgemäßen Methanolsynthese können in dem Destillationsschritt Wasser über Sumpf und die leichtflüchtigen Komponenten über Kopf abgetrennt werden. Das so gewonnene Methanol kann dann zum Beispiel einem Ottokraftstoff zugegeben werden. Soll hochreines Methanol hergestellt werden, ist es vorteilhaft zwei oder mehrere Destillationskolonnen einzusetzen.

Eine andere bevorzugte Weiterbildung der vorliegenden Erfindung sieht vor, dass ein bei der Methanolsynthesereaktion erhaltener Produktstrom zunächst einem Hochdruckabscheider zugeführt und ein in diesem Hochdruckabscheider von einem Methanol-haltigen Produktstrom abgetrennter Gasstrom teilweise als Kreisgas in die Methanolsynthereaktion rückgeführt und ein weiterer Teilstrom aus dem Hochdruckabscheider dem Destillationsschritt zugeführt wird.

Gemäß einer bevorzugten Fortbildung der vorgenannten Variante kann beispielsweise ein bei einer Methanolsynthesereaktion erhaltener gasförmiger Produktstrom zunächst nach Abkühlung einem Hochdruckabscheider zugeführt werden, in dem ein erster Gasstrom von einem flüssigen Methanol- haltigen Produktstrom abgetrennt wird und der Methanol-haltige Produktstrom danach unter Druckreduzierung einem Niederdruckabscheider zugeführt werden, in dem ein zweiter Gasstrom von dem Methanol-haltigen Produktstrom abgetrennt wird, wobei der erste Gasstrom mindestens teilweise als Kreisgas in die Methanolsynthesereaktion rückgeführt, der zweite Gasstrom anschließend dem Destillationsschritt zugeführt wird und der übrige Methanol-haltige Produktstrom ebenfalls dem Destillationsschritt zugeführt wird.

Durch die Rückführung des Kopfprodukts aus dem Destillationsschritt in eine Misch- und Verdichtersektion ergeben sich in vorteilhafter Weise eine signifikante Minimierung der Abgasmenge und eine Erhöhung des Gesamtwirkungsgrads der Anlage.

Durch den Abzug eines Teilstroms vom Kreisgas zur Destillationsvorrichtung kann zum einen ein konstanter Strom über einen Recycle-Kompressor zum Reaktor zurückgeführt werden. Zum anderen kann durch die Aufteilung des Fluidstroms das H₂ zu CO₂ Verhältnis in bestimmten Grenzen eingestellt werden. Bei Wegfall dieses Teilstroms ist es vorteilhaft, einen Teil des Recycle-Stroms (des Stroms von der Destillationsvorrichtung zur Misch- und Verdichtersektion) abzuführen. Möglich ist es auch an beiden Stellen jeweils einen kleinen Abzug (Abgasstrom) vorzusehen.

Ein kleiner Abgasstrom kann anfallen, wenn ein Katalysator mit schlechter Selektivität eingesetzt wird, wodurch sich vermehrt Nebenprodukte bilden, oder wenn ungünstige Betriebsparameter (beispielsweise hohe Temperaturen) bei schon fortgeschrittener Deaktivierung des Katalysators gewählt werden und so verstärkt Nebenprodukte gebildet werden.

Sofern sich bei dem erfindungsgemäßen Verfahren zu reinigende Abgasströme ergeben, die trotz kleiner Mengen der katalytischen Abgasreinigung zugeführt werden, können diese beispielsweise Kohlenmonoxid und/oder Kohlendioxid und/oder Wasser enthalten, sowie Reste von Methanol, Wasserstoff und Nebenprodukte wie beispielsweise Methylformiat und Dimethylether.

Die Methanolsynthese nach dem erfindungsgemäßen Verfahren erfolgt vorzugsweise bei Temperaturen im Bereich von etwa 200 °C bis etwa 300 °C.

Gegenstand der vorliegenden Erfindung ist weiterhin eine Anlage zur Herstellung von Methanol durch Umsetzung von Kohlendioxid mit Wasserstoff, umfassend wenigstens eine Reaktoreinheit für die Methanolsynthese sowie umfassend wenigstens eine der Reaktoreinheit nachgeschaltete erste Trennvorrichtung zur Abtrennung leichtflüchtiger und/oder flüssiger Bestandteile von einem Methanol- haltigen Produktstrom, wobei die Anlage weiterhin wenigstens eine der ersten Trennvorrichtung nachgeschaltete weitere Trennvorrichtung zur Abtrennung leichtflüchtiger Bestandteile durch Destillation oder zur Abtrennung von Wasser durch Kondensation aufweist, wobei wenigstens eine mit der Trennvorrichtung verbundene Rückführleitung für eine zumindest teilweise Rückführung eines in der Trennvorrichtung abgetrennten Gasstroms in einen Bereich stromaufwärts der Reaktoreinheit vorgesehen ist und wenigstens eine mit der Trennvorrichtung verbundene Abgasleitung zur teilweisen oder vollständigen Abführung eines Abgasstroms aus der Anlage vorgesehen ist. Vorzugsweise umfasst die erfindungsgemäße Anlage weiterhin eine Elektrolysevorrichtung zur Erzeugung von Wasserstoff aus Wasser, sowie Mittel zur Zuführung des bei der Elektrolyse erzeugten Wasserstoffs zu der Reaktoreinheit zur Herstellung von Methanol. In der Elektrolysevorrichtung kann die zum Beispiel aus Stromspitzen stammende elektrische Energie zur Elektrolyse von Wasserstoff aus Wasser genutzt werden. Da sich der Wasserstoff nicht gut speichern lässt, ist es vorteilhaft, den Wasserstoff in einen anderen Energieträger umzuwandeln, wozu man diesen gemäß der Erfindung bevorzugt mit Kohlendioxid zu Methanol umsetzt. Das Methanol lässt sich in geeigneten Tanks speichern und kann als Energieträger zu einem gegebenen Zeitpunkt genutzt werden oder aber als reaktive Komponente zur Herstellung von weiteren chemischen Grundstoffen eingesetzt werden.

Sofern es gemäß einer der zuvor genannten Verfahrensvarianten Abgasströme gibt, die aus dem System abgeführt werden müssen, umfasst die erfindungsgemäße Anlage vorzugsweise weiterhin wenigstens eine Einrichtung zur katalytischen Abgasreinigung eines aus der Anlage abzuführenden Abgas- und/oder Purgegasstroms, welche mit der Reaktoreinheit über die Abgasleitung mittelbar oder unmittelbar in Wirkverbindung steht.

Vorzugsweise umfasst diese Anlage weiterhin wenigstens einen zwischen der Elektrolysevorrichtung und der Reaktoreinheit angeordneten Verdichter, mittels dessen der Eduktgasstrom auf den für die Reaktoreinheit notwendigen Eingangsdruck gebracht werden kann.

Vorzugsweise umfasst die erfindungsgemäße Anlage weiterhin als erste Trennvorrichtung wenigstens einen der Reaktoreinheit nachgeschalteten Hochdruckabscheider zur Abtrennung gasförmiger Bestandteile von einem Methanol-haltigen Produktstrom. In dem Hochdruckabscheider können durch eine vorgeschaltete Kühlung und hohen Druck flüssige Komponenten wie beispielsweise Methanol und Wasser von leichtflüchtigen Komponenten abgetrennt werden. Als Kühlmedium kann beispielsweise das im CO₂-Verdampfer abgekühlte Kühlmedium eingesetzt werden. Eine Kühlung mit Wasser oder Luft ist prinzipiell ebenfalls möglich.

Vorzugsweise umfasst die erfindungsgemäße Anlage weiterhin als Trennvorrichtung wenigstens einen der Reaktoreinheit nachgeschalteten, vorzugsweise einem Hochdruckabscheider nachgeschalteten Niederdruckabscheider zur Abtrennung flüssiger und/oder gasförmige Bestandteile von einem Methanol-haltigen Produktstrom. In dem Niederdruckabscheider wird das Fluidgemisch entspannt und es wird eine Gasphase erzeugt, die dann zum Beispiel entweder vollständig oder auch nur teilweise der Destillationsvorrichtung zugeführt werden kann. Bei der letztgenannten Variante kann ein Teilstrom aus dem Niederdruckabscheider als Abgas, gegebenenfalls nach katalytischer Reinigung, aus dem System abgeführt werden. Eine solche Abführung von Abgas kann aber alternativ auch nach der Destillationsvorrichtung erfolgen, die erfindungsgemäß bevorzugt als weitere Trennvorrichtung verwendet wird.

Um die in der Destillationsvorrichtung abgetrennten Komponenten erneut der Methanolsynthese zuzuführen, umfasst die erfindungsgemäße Anlage bevorzugt wenigstens eine Rückführleitung für die Rückführung eines in der Destillationsvorrichtung abgetrennten Gasstroms in einen Bereich stromaufwärts der Reaktoreinheit.

Sofern ein Abführen eines Teilstroms als Abgas aus dem Prozess vorgesehen ist, kann dies auch beispielsweise nach dem Trennvorgang im Hochdruckabscheider erfolgen, so dass in diesem Fall die Anlage vorzugsweise wenigstens eine Gasleitung von dem Hochdruckabscheider zu der Einrichtung zur katalytischen Abgasreinigung umfasst. Alternativ dazu kann auch ein Abführen eines Teilstroms als Abgas nach dem Trennvorgang im Niederdruckabscheider vorgesehen sein, so dass dann vorzugsweise wenigstens eine Gasleitung von dem Niederdruckabscheider zu der Einrichtung zur katalytischen Abgasreinigung vorgesehen ist. Oder aber das Abführen eines Teilstroms als Abgas oder Purgegas erfolgt nach der Destillation, so dass dann vorzugsweise wenigstens eine Gasleitung vorgesehen ist, die von der Destillationsvorrichtung zu der katalytischen Abgasreinigung führt. Durch Rückführung der in der Destillation abgetrennten Komponenten und die erfindungsmäßige Verschaltung der Gasströme aus den Abscheidern werden mögliche Abgasströme soweit minimiert, dass eine Abgasreinigung nicht unbedingt erforderlich ist. Allerdings ist es von Vorteil, eine geeignete Position für ein Abblasen an die Umgebung sicherzustellen.

Gemäß einer bevorzugten Weiterbildung der vorliegenden Erfindung wird ein Kreisgas von dem Hochdruckabscheider ausgehend in die Methanolsynthese zurückgeführt. Bei dieser Variante ist somit wenigstens eine von dem Hochdruckabscheider ausgehende Rückführleitung für Gase zum Eingangsbereich der Reaktoreinheit vorgesehen, wobei die Anlage dann vorzugsweise weiterhin einen im Strömungsweg zwischen dem Hochdruckabscheider und einem Eingangsbereich der Reaktoreinheit, vorzugsweise in der Rückführleitung, angeordneten Verdichter umfasst.

Man kann auch bei der vorgenannten Variante vorsehen, dass ein Teilstrom des Kreisgases aus dem Hochdruckabscheider abgetrennt und in die Destillationsvorrichtung geleitet wird. Bei dieser bevorzugten Variante ist eine von dem Hochdruckabscheider ausgehenden Rückführleitung abzweigende Zweigleitung für die Zuführung mindestens eines Teilstroms der im Hochdruckabscheider abgetrennten leichtflüchtigen Bestandteile in den oberen Bereich der Destillationsvorrichtung vorgesehen. Diese Zweigleitung kann dann wiederum in eine Leitung einmünden, welche der Zuführung von im Niederdruckabscheider abgetrennten leichtflüchtigen Bestandteilen zum oberen Bereich der Destillationsvorrichtung dient.Eine weitere mögliche Variante der Erfindung sieht vor, dass Wasserstoff in einem Wasserstoffspeicher gespeichert wird, der bevorzugt nach einem oder mehreren Verdichtern und vor der Reaktoreinheit für die Methanolsynthese angeordnet ist. Damit könnte ein Stromausfall für eine gewisse Zeit überbrückt werden, und so die Kapazität der Anlage geregelt angepasst werden und auf Teillast weiterbetrieben werden. Für den Wasserstoffspeicher könnte auch ein separater Verdichter vorgesehen werden, um den Wasserstoff bei einem hohen Druck zu speichern und dann bei Bedarf in die Verdichterstrecke zuzugeben.

Nachfolgend werden einige bevorzugte Betriebsbedingungen für die Methanolsynthese nach dem erfindungsgemäßen Verfahren aufgeführt. Die Reaktion im Methanolsynthesereaktor erfolgt vorzugsweise bei einer Temperatur im Bereich von 200 °C bis 300 °C, insbesondere bei 210 °C bis 280 °C und bei einem Druck von vorzugsweise 30 bis 100, insbesondere bei 40 bis 100 bara (bar absolut). Hohe Temperaturen können zu einer verstärkten Deaktivierung des Katalysators sowie zu vermehrter Bildung von Nebenprodukten führen.

Über das molare Verhältnis der beiden Reaktanden Wasserstoff : Kohlendioxid am Reaktoreingang lässt sich die Methanolsynthese gezielt steuern und die Bildung von Nebenprodukten minimieren. Das stöchiometrische Verhältnis für die Synthese von Methanol aus Wasserstoff und Kohlendioxid liegt bei 3 : 1. Jedoch kann man bevorzugt ein überstöchiometrisches Verhältnis von H₂ zu CO₂ am Reaktoreingang im Bereich von 5 bis 12 wählen, auch um die Deaktivierung des Katalysators abzuschwächen.

Für den Methanolsynthesereaktor kommt im Prinzip jeder Reaktortyp in Betracht. Beispielsweise kann dies ein wassergekühlter Rohrbündelreaktor sein.

Für die erfindungsgemäße Methanolsynthese kommen grundsätzlich die dem Fachmann für die Reaktion bekannten Katalysatoren in Betracht. Hinsichtlich des Katalysators für diese Reaktion gibt es keine spezifischen Einschränkungen. Nur beispielhaft seien Kupfer-basierte Katalysatoren genannt.

Die in den Unteransprüchen genannten Merkmale betreffen bevorzugte Weiterbildungen der vorliegenden Erfindung. Weitere Vorteile ergeben sich aus der nachfolgenden Detailbeschreibung.

Nachfolgend wird die vorliegende Erfindung anhand von Ausführungsbeispielen unter Bezugnahme auf die beiliegenden Zeichnungen näher erläutert. Dabei zeigen:
Figur 1 ein schematisches Fließbild einer ersten beispielhaften Anlage zur Herstellung von Methanol mit Verbrennung eines Teils der Purgegase/Abgase;
Figur 2 ein schematisches Fließbild einer zweiten beispielhaften Anlage zur Herstellung von Methanol mit katalytischer Verbrennung eines Teils der Purgegase/Abgase;
Figur 3 ein schematisches Fließbild einer dritten beispielhaften Anlage zur Herstellung von Methanol;
Figur 4 ein schematisches Fließbild einer vierten beispielhaften Anlage zur Herstellung von Methanol mit Abführung eines Teils der Purgegase/Abgase;
Figur 5 ein schematisches Fließbild einer fünften beispielhaften Anlage zur Herstellung von Methanol ohne Abführung eines Abgasstroms;
Figur 6 ein schematisches Fließbild einer sechsten beispielhaften Anlage zur Herstellung von Methanol mit Zufuhr weiterer Eduktgase zur Methanolsynthese;
Figur 7 ein schematisches Fließbild einer siebten beispielhaften Anlage zur Herstellung von Methanol mit einer vereinfachten Anlagenkonfiguration.

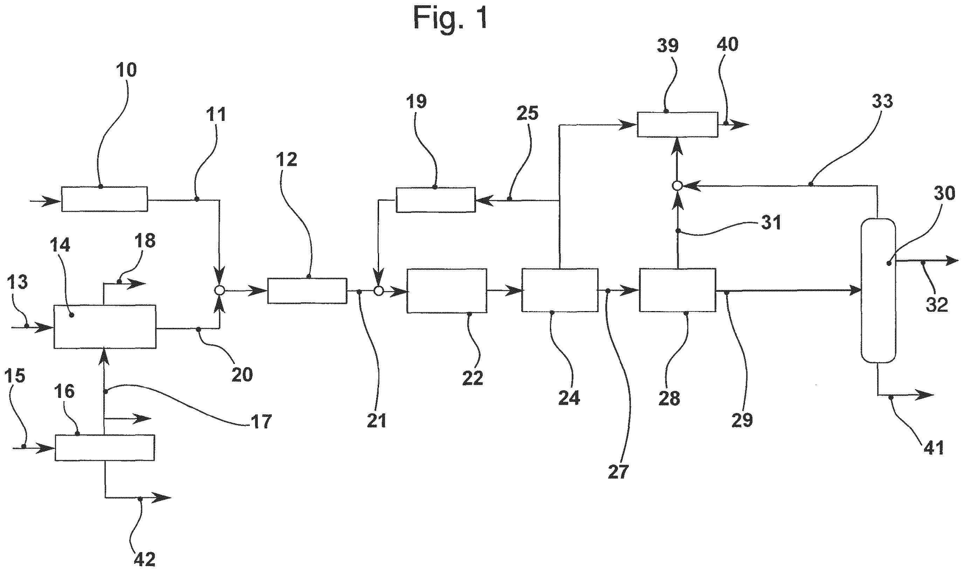

Nachfolgend wird zunächst auf die Figur 1 Bezug genommen. Es handelt sich in dem Ausführungsbeispiel insbesondere um eine so genannte "Small-scale"-Anlage zur Methanolherstellung mit einer Kapazität in der Größenordnung von beispielsweise bis zu mehreren 10.000 t/a. Es ist eine externe Quelle für Kohlendioxid vorgesehen, welches beispielsweise kryogen vorliegen kann und einem Verdampfer 10 zugeführt wird, von wo aus das CO₂ über eine Leitung 11 in einen Verdichter 12 gelangt. Der zweite Ausgangsstoff für die Methanolherstellung ist Wasserstoff, welcher elektrolytisch aus Wasser gewonnen wird. Dazu wird vorzugsweise aus regenerativen Energien gewonnener Strom 13 eingesetzt, um die Elektrolysevorrichtung 14 zu betreiben. Das Edukt für die Elektrolyse ist Wasser, welches über eine Leitung 15 zunächst gegebenenfalls in eine Aufbereitungsvorrichtung 16 geleitet wird, in der zum Beispiel eine Umkehrosmose und/oder ein lonenaustausch vorgesehen ist. Von dort aus gelangt das aufbereitete Wasser über die Leitung 17 als Edukt in die Elektrolysevorrichtung 14. An dieser Stelle kann beispielsweise ein Teilstrom an Frischwasser für ein mögliches Dampf- und Kondensatsystem und/oder und einen geschlossenen Kühlmediumkreislauf abgezweigt werden. Das Abwasser aus der Aufbereitungsvorrichtung kann im einfachsten Fall über die Abwasserleitung 42 aus dem System abgeführt werden.

Bei der Elektrolyse von Wasser entsteht neben dem Wasserstoff auch Sauerstoff, welcher aus der Elektrolysevorrichtung 14 über eine Leitung 18 abgeleitet werden kann und entweder bei der katalytischen Nachverbrennung der Purgegase/Abgase als zusätzlicher Brennstoff eingesetzt wird oder aber einem anderen Verwendungszweck außerhalb des Systems zugeführt wird. Der bei der Elektrolyse erzeugte Wasserstoff wird über die Leitung 20 dem Verdichter 12 zugeführt, dem auch das CO₂ über die Leitung 11 zugeführt wird. Von dem Verdichter 12 aus wird dann das vereinigte Eduktgemisch aus CO₂ und H₂ über die Leitung 21 in den Methanolsynthesereaktor 22 eingespeist.

In dem Methanolsynthesereaktor 22 erfolgt die Methanolsynthese und der diesen Reaktor verlassende Produktstrom wird über die Leitung 23 einem Hochdruckabscheider 24 zugeführt. Von diesem aus kann eine Rückführleitung 25 zu einem Verdichter 19 vorgesehen sein, in dem ein edukthaltiges Gasgemisch, welches in dem Hochdruckabscheider 24 von dem Produktstrom abgetrennt wurde, verdichtet und nach Verdichtung zurückgeführt und wieder dem Methanolsynthesereaktor 22 zugeleitet wird. Dieser Methanolsynthesereaktor 22 arbeitet im vorliegenden Beispiel bei einer erhöhten Temperatur, beispielsweise in einer Größenordnung von 200 °C bis 300 °C und bei einem erhöhten Druck, welcher zum Beispiel in einem Bereich von etwa 30 bar bis 100 bar liegen kann. Außerdem wird für die Methanolsynthese in der Regel ein Katalysator verwendet. Der Methanol-haltige Produktstrom, der den Methanolsynthesereaktor 22 verlässt, wird einem Hochdruckabscheider 24 zugeführt, verlässt diesen über die Leitung 27, wird dann gegebenenfalls einem Niederdruckabscheider 28 zugeführt, in dem eine weitere Abtrennung von Gasen von dem Methanol-haltigen Produktstrom erfolgt.

Eine weitere Reinigung dieses Rohmethanols erfolgt in einer mit dem Niederdruckabscheider 38 über eine Leitung verbundenen Destillationsvorrichtung 30, in der zum einen eine Abtrennung von leichtflüchtigen Komponenten erfolgt, die vom Kopf der Destillationsvorrichtung 30 über eine Leitung 33 abgeleitet werden und im einfachsten Fall gegebenenfalls als Abgas aus dem System abgeführt werden können. Weiterhin ist eine zweite vom Niederdruckabscheider 28 ausgehende Leitung 31 vorgesehen, über die dort abgetrennte leichtflüchtige Komponenten ebenfalls dem Abgasstrom zugeführt werden können. Diese leichtflüchtigen Komponenten können insbesondere bei Anfall geringer Mengen über eine Fackel 39 verbrannt und das Abgas kann über die Leitung 40 aus dem System abgeführt werden.

Neben den leichtflüchtigen Komponenten kann in der Destillationsvorrichtung 30 Wasser über Sumpf von dem Methanol abgetrennt und über die Leitung 41 kann das Abwasser bei dieser einfachen Anlage aus dem System abgeführt werden. Das Methanol wird dann in einem hohen Reinheitsgrad über die Leitung 32 aus dem System ausgeschleust und kann beispielsweise in Tanks gespeichert werden.

Ein Teil der im Hochdruckabscheider 24 abgetrennten leichtflüchtigen Komponenten wird als Kreisgas über die Rückführleitung 25 einem weiteren Verdichter 19 zugeführt und dann von dort aus in die Leitung 21 für Edukte eingespeist, so dass diese Komponenten in den Methanolsynthesereaktor 22 zurückgeführt werden können.

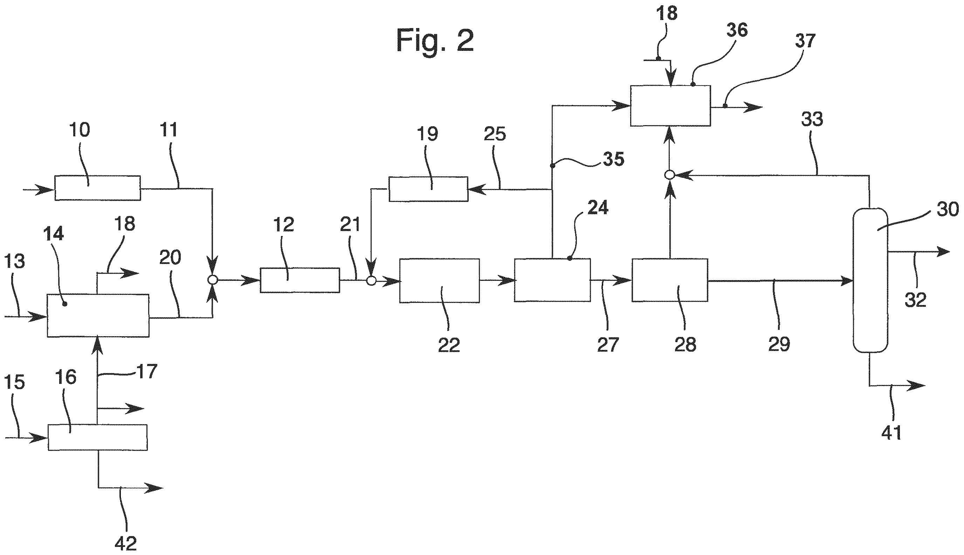

Nachfolgend wird unter Bezugnahme auf Figur 2 ein zweites Ausführungsbeispiel einer erfindungsgemäßen Anlage zur Herstellung von Methanol beschrieben. Der Aufbau ist ähnlich wie bei dem zuvor anhand von Figur 1 beschriebenen Ausführungsbeispiel, denn auch hier handelt es sich um eine kleinere Anlage (small-scale) in einer Basiskonfiguration, wobei allerdings im Unterschied zu dem Beispiel von Figur 1 eine Abgasreinigung vorgesehen ist. Alle Anlagenbauteile, die dem Aufbau gemäß Figur entsprechen, werden hier nicht noch einmal erläutert. Insoweit wird auf die obigen Ausführungen verwiesen. Der Unterschied zu dem Beispiel von Figur 1 besteht darin, dass anstelle der Fackel 39 eine Einrichtung 36 für die katalytische Abgasreinigung vorgesehen ist.

Über die Gasleitung 33 (siehe Figur 1) wird der den Kopf der Destillationsvorrichtung 30 verlassende Gasstrom einer katalytischen Nachverbrennung 36 zugeführt. Der im Hochdruckabscheider 24 abgetrennte Anteil an leichtflüchtigen Komponenten wird als Purgegasstrom dem Kreislauf entzogen und über die Leitung 35 der Einrichtung 36 zur katalytischen Nachverbrennung zugeführt. Diese kann mit Sauerstoff über die Leitung 18 gespeist werden, welcher als Nebenprodukt in der Elektrolysevorrichtung 14 anfällt, so dass je nach Zusammensetzung der Purgegase der Sauerstoff als zusätzlicher Brennstoff zugeführt dienen kann, um die Verbrennung zu fördern. In der Einrichtung 36 erfolgt die katalytische Nachverbrennung, so dass ein gereinigter Abgasstrom erzeugt wird, welcher die Anlage über die Ausgangsleitung 37 verlässt.

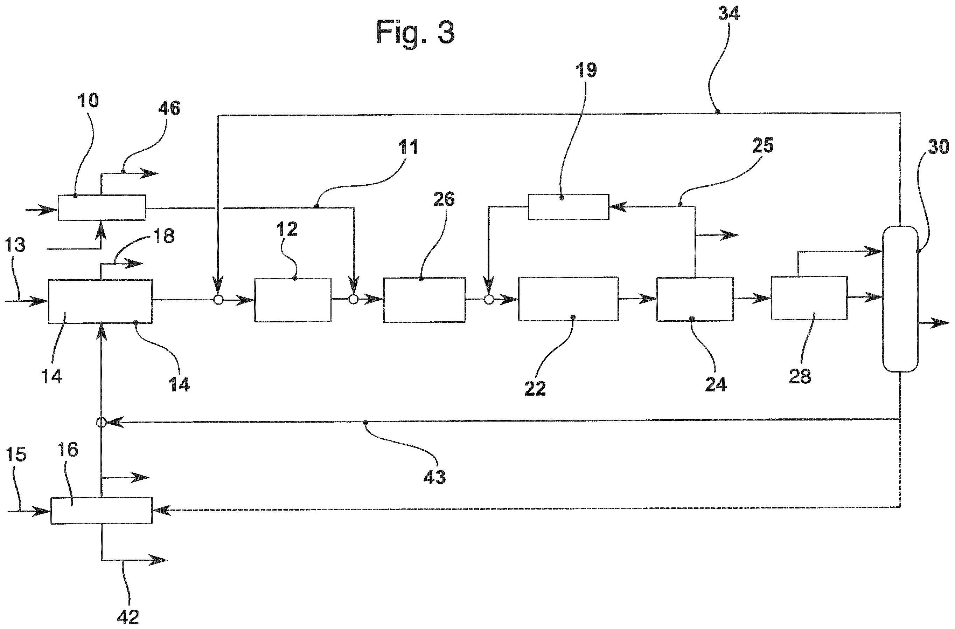

Nachfolgend wird unter Bezugnahme auf die Figur 3 ein drittes Ausführungsbeispiel der vorliegenden Erfindung erläutert. Diese Variante unterscheidet sich von den beiden zuvor beschriebenen Ausführungsbeispielen zum einen dadurch, dass der Anfall von Abgas minimiert wurde. Der Fluidstrom der in der Destillationsvorrichtung 30 abgetrennten leichtflüchtigen Komponenten wird hier als Recycle-Strom über die Rückführleitung 34 in einen Bereich zurückgeführt, der vor einem ersten Verdichter oder einer ersten Verdichterstufe 12 und stromaufwärts des Methanolsynthesereaktors 22 liegt. Anders als bei den zuvor beschriebenen Varianten ist hier ein erster Verdichter (Verdichterstufe) 12 vorgesehen, dem zum einen der Wasserstoff aus der Elektrolyse 14 zugeführt wird und zusätzlich dieser Recyclestrom. Das den ersten Verdichter 12 verlassende Gemisch wird dann zusammen mit dem aus dem Verdampfer 10 über die Leitung 11 zugeführten Kohlendioxid einem weiteren Verdichter oder einer weiteren Verdichterstufe 26 zugeführt, da eine mehrstufige Verdichtung notwendig ist, um die Eduktgase auf den für die Methanolsynthese vorrgesehenen Druck zu bringen. Vom Ausgang des weiteren Verdichters 26 gelangt der Eduktstrom dann in den Methanolsynthesereaktor 22, wobei diesem zusätzlich das vom Hochdruckabscheider 24 über die Rückführleitung 25 rückgeführte Kreisgas zugeführt wird, nachdem dieses in dem Verdichter 19 verdichtet wurde.

Weiterhin wird bei dieser Variante auch das bei der Destillation im Sumpf anfallende Wasser nicht aus dem System abgeführt, sondern im Kreislauf über die Rückführleitung als Prozesswasser zurück in die Wasserelektrolyse 14 geleitet. Gegebenenfalls kann dieses bei der Destillation abgetrennte Wasser auch zunächst aufbereitetwerden, beispielsweise zur Entfernung von Methanol durch ein Membranverfahren. Gegebenenfalls kann man auch im Wasser gelöste Substanzen oder andere Verunreinigungen durch geeignete Verfahren abtrennen. Wenn keine Aufbereitung des bei der Destillation abgetrennten Wassers notwendig ist, kann bei dieser Variante des Verfahrens die Reaktion geführt werden, ohne dass Abwasser anfällt. Sofern eine Aufbereitung des Wassers notwendig ist, wird das Abwasser aus der Aufbereitung 16 über die Abwasserleitung 42 aus dem System abgeführt.

Ein weiterer Unterschied liegt bei der Variante gemäß Figur 3 darin, dass man Kühlmedium aus einem geschlossenen Kühlkreislauf 46 verwendet, um das kryogene CO₂ im Verdampfer 10 zu verdampfen und zu erwärmen, bevor man es über die Leitung 11 der zweiten Verdichterstufe 26 zuführt. Als Kühlmittel kann ein Glykol/Wasser-Gemisch verwendet werden. Das aufgeheizte Kühlmedium muss man so nicht separat kühlen, sondern kann die im Kühlmedium enthaltene Wärme für die CO₂-Verdampfung nutzen.

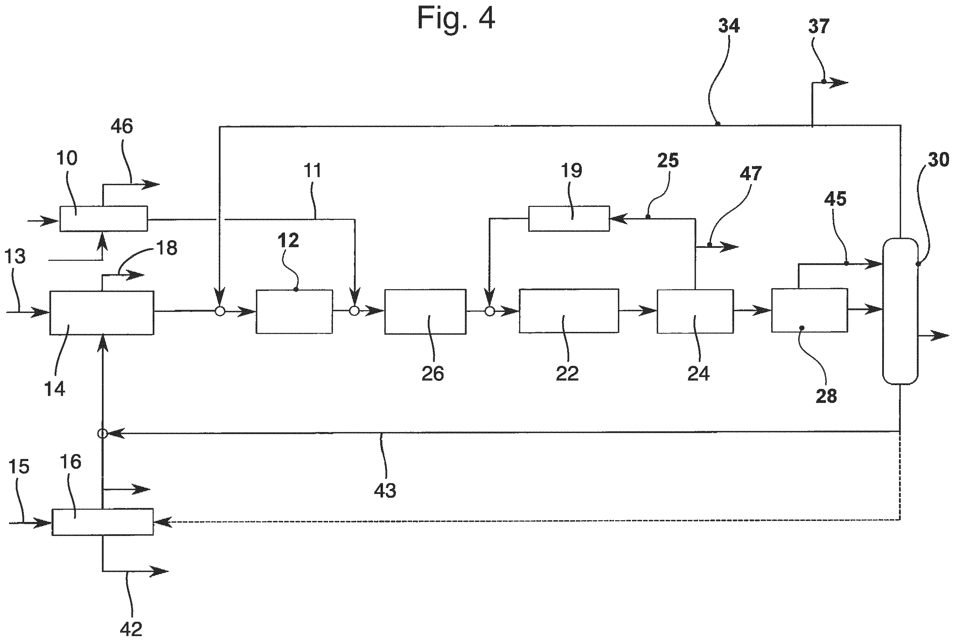

Nachfolgend wird unter Bezugnahme auf Figur 4 ein viertes Ausführungsbeispiel der vorliegenden Erfindung erläutert. Diese Anlagenkonzeption für die Methanolsynthese entspricht im Wesentlichen derjenigen, die zuvor unter Bezugnahme auf Figur 3 beschrieben wurde, mit dem Unterschied, dass bei der Variante gemäß Figur 4 ein Abgasstrom vorgesehen ist, der aus dem System abgeführt wird. Aus der Rückführleitung 34, die vom Kopf der Destillationsvorrichtung 30 ausgehend den Recyclestrom in den Bereich vor dem ersten Verdichter (oder der Verdichterstufe) 12 zurückführt, wird ein Teilstrom über eine Abgasleitung 37 abgezweigt und aus dem System abgeführt. Die Abgasmenge kann jedoch minimiert werden, da bei der Variante gemäß Figur 4 ebenso wie bei derjenigen gemäß Figur 3 die Gasphase aus dem Niederdruckabscheider 28 über eine weitere Leitung 45 direkt in die Destillationsvorrichtung (Destillationskolonne) 30 zugegeben wird. Der Gesamtwirkungsgrad der Anlage wird dadurch erhöht. Die Variante gemäß Figur 4 offenbart auch die Möglichkeit, zusätzlich einen kleinen Abgasstrom 47 vorzusehen, der aus der Leitung 25 abgezweigt wird, über die das Kreisgas in die Methanolsynthese zurückgeführt wird. Mit dem Abgasstrom 47 wird der Kreisgasstrom nicht zu groß und das Verhältnis H₂ zu CO₂ in den Reaktor kann in gewissen Grenzen eingestellt werden. Weitere Stellgröße zur Einstellung des gewünschten H₂ zu CO₂-Verhältnisses ist die Zugabe der beiden Edukte CO₂ und H₂ vor der Misch- und Verdichtersektion.

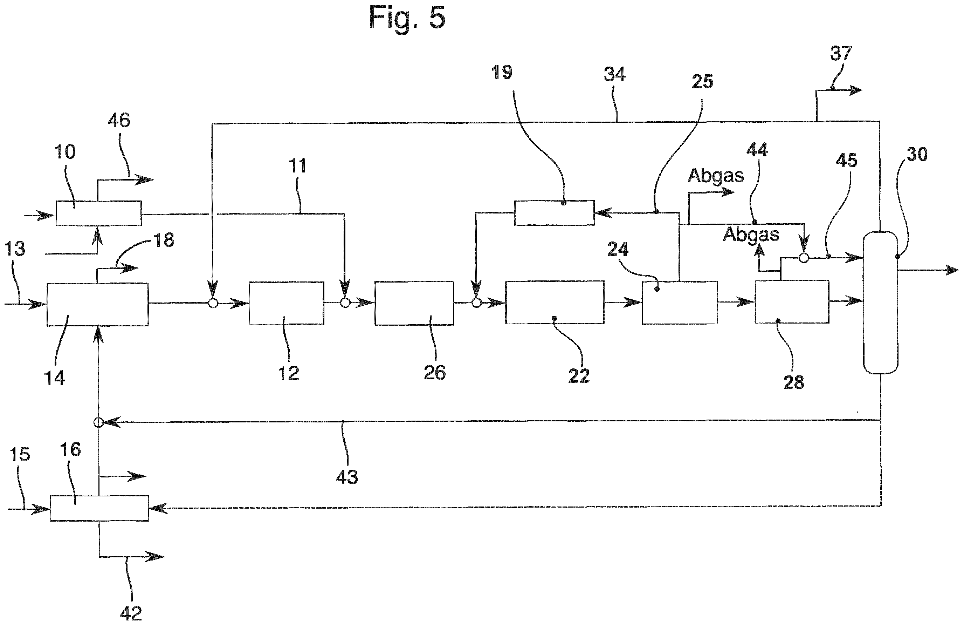

Nachfolgend wird unter Bezugnahme auf Figur 5 ein fünftes Ausführungsbeispiel der vorliegenden Erfindung erläutert. Diese Anlagenkonzeption für die Methanolsynthese stellt eine Variante dar, bei der kein oder ein minimaler Strom an Abgas aus dem System abgeleitet wird. Von der zuvor beschriebenen Variante gemäß Figur 3 unterscheidet sie sich dadurch, dass von dem Kreisgas, welches vom Hochdruckabscheider 24 ausgehend über die Rückführleitung 25 und den Verdichter 19 in die Methanolsynthese 22 zurückgeführt wird, ein Teilstrom über eine Leitung 44 abgezweigt wird, welche in die weitere Leitung 45 einmündet. Eine Alternative dazu wäre die direkte Zufuhr in die Destillationsvorrichtung 30. Auf diese Weise kann dieser Teilstrom dem Gasstrom zugemischt werden, welcher vom Niederdruckabscheider 28 direkt zur Destillationsvorrichtung 30 führt. Durch den Abzug eines Teilstroms vom Kreisgas kann zum einen ein konstanter Strom über den Recycle-Verdichter 19 zum Methanolreaktor 22 zurückgeführt werden. Durch die Aufteilung des Stroms wird eine Flexibilität bezüglich der rückgeführten Menge geschaffen.

Die bei den Varianten gemäß den Figuren 3 bis 7 vorgesehene Rückführleitung 34 führt den Gasstrom aus der Destillationsvorrichtung 30 zurück vor den Verdichter 12, mittels dessen das Einsatzgas verdichtet wird. Der Gasstrom aus dem Niederdruckabscheider 28 wird in die Destillationsvorrichtung 30 geführt. Ein Teilstrom des Gasstroms aus dem Hochdruckabscheider 24 kann, wie dies in den Ausführungsbeispielen gemäß den Figuren 5 bis 7 dargestellt ist, ebenfalls über die Leitungen 44 und 45 der Destillationsvorrichtung 30 zugeführt werden. Alternativ dazu kann aber der Gasstrom aus dem Hochdruckabscheider 24 entweder vollständig wie bei den Varianten gemäß den Figuren 3 und 4 oder teilweise, wie bei den Varianten gemäß den Figuren 5 bis 7, gegebenenfalls unter Verdichtung über den weiteren Verdichter 19, zum Eingang des Methanolsynthesereaktors 22 zurückgeführt werden. Durch diese Maßnahmen wird die Effizienz der erfindungsgemäßen Methanolanlage signifikant verbessert.

Die in den Ausführungsbeispielen gemäß den Figuren 3 bis 5 dargestellte integrierte Verschaltung der Methanolanlage hat den Vorteil, dass sich die bei dem Verfahren anfallende Abgasmenge erheblich reduzieren lässt. Außerdem können die Einsatzmengen der Eduktgase Kohlendioxid und Wasserstoff bei gleichbleibender produzierter Methanolmenge erheblich, beispielsweise um einige Prozentpunkte, reduziert werden. Damit wird auch die bei der Elektrolyse zur Gewinnung des Wasserstoffs erforderliche Strommenge gesenkt.

Der in den Ausführungsbeispielen gemäß den Figuren 2 bis 6 dargestellte Verdampfer 10 für das Verdampfen des kryogen vorliegenden Kohlendioxids erzeugt Kälte, welche beispielsweise zur Kühlung des Wasserstoffstromes aus der Elektrolyse verwendet werden kann. Der Wasserstoffstrom lässt sich so auf Temperaturen im Bereich von beispielsweise weniger als 5 °C herunterkühlen. Dadurch kann ein hoher Anteil an Wasser rückgewonnen werden, welcher dann wieder in die Elektrolysevorrichtung 14 für die Wasserelektrolyse geleitet werden kann. Als Kühlmedium dient hier beispielsweise ein Glykol/Wasser-Gemisch.

Weiterhin kann in vorteilhafter Weise das in dem Prozess als Koppelprodukt anfallende Prozesswasser beispielsweise über die in Figur 3 dargestellte Rückführleitung 43 zur Elektrolysevorrichtung 14 zurückgeführt werden.

Weiter Vorteile des Verfahrens ergeben sich aus der oben anhand von Figur 2 beschriebenen Einrichtung 36 zur katalytischen Abgasreinigung.

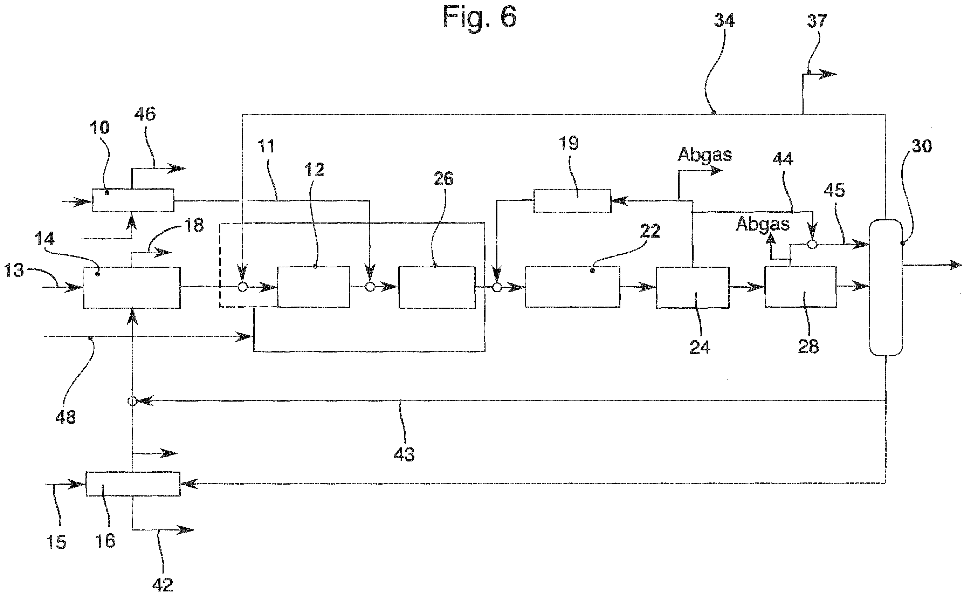

Nachfolgend wird unter Bezugnahme auf Figur 6 ein sechstes Ausführungsbeispiel der vorliegenden Erfindung erläutert. Diese Anlagenkonzeption für die Methanolsynthese sieht das Abführen eines kleinen Abgasstroms 37 aus der Rückführleitung 34 vor, über die der Recyclestrom in den Bereich vor den Verdichter 12 zurückgeführt wird. Ein solcher kleiner Abgasstrom 37 kann beispielsweise dann anfallen, wenn ein Katalysator mit schlechter Selektivität eingesetzt wird oder ungünstige Betriebsparameter bei fortgeschrittener Deaktivierung des Katalysators gewählt und so verstärkt Nebenprodukte gebildet werden.

Die Ausführungsform gemäß Figur 6 unterscheidet sich von den zuvor beschriebenen Varianten weiterhin dadurch, dass in die Misch- und Verdichtersektion, die die beiden Verdichter 12 und 26 bzw. Verdichterstufen umfasst, von außerhalb des Systems weitere Eduktgase zugegeben werden. Dazu ist eine weitere Leitung 48 vorgesehen, die von außerhalb zu der Misch- und Verdichtersektion führt, über die CO, H₂ oder ein Synthesegas umfassend CO, CO₂ und H₂ zugemischt werden kann. Diese weiteren Gase werden in der Misch- und Verdichtersektion mit dem Wasserstoff aus der Elektrolyse 14 und/oder dem Kohlendioxid aus dem Verdampfer gemischt und das Gemisch wird dann dem Methanolsynthesereaktor 22 zugeleitet.

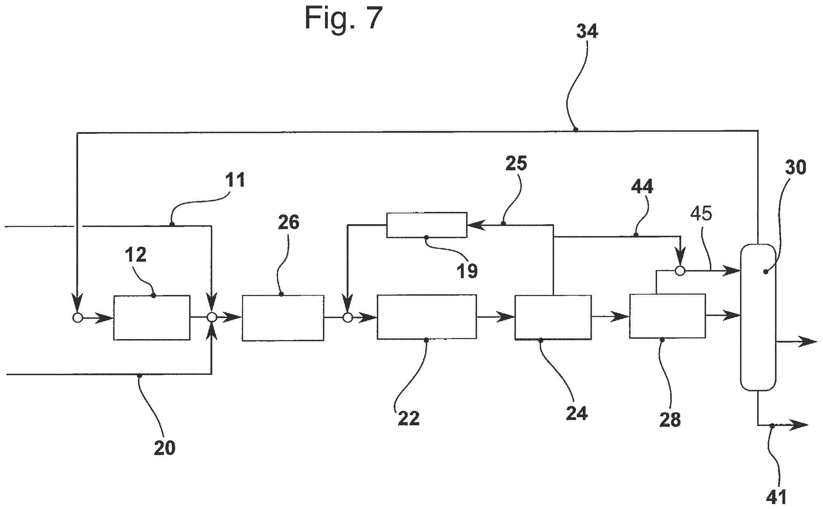

Nachfolgend wird unter Bezugnahme auf Figur 7 ein siebtes Ausführungsbeispiel der vorliegenden Erfindung erläutert. Diese Variante betrifft eine etwas vereinfachte Anlagenkonzeption für die Methanolsynthese. Hier wird Kohlendioxid von außerhalb des Systems in gasförmigem Zustand über die Leitung 11 zugeführt in einen Bereich, der stromabwärts des ersten Verdichters bzw. der ersten Verdichterstufe 12 angeordnet ist. Der Wasserstoff wird nicht im System durch Elektrolyse erzeugt, sondern auch von außerhalb über die Leitung 20 zugeführt, was beispielsweise dann in Betracht kommt, wenn an einem Standort Wasserstoff zur Verfügung steht. Es sind auch beliebige andere Kombinationen für die Bereitstellung der Eduktgase möglich, beispielsweise die Verwendung von H₂ aus der Elektrolyse in Verbindung mit gasförmigem CO₂ oder beispielsweise die Verwendung von H₂ von außerhalb in Verbindung mit kryogenem CO₂. Auch in Figur 7 kann analog wie oben bei Figur 6 beschrieben ein Abgasstrom 37 vorgesehen sein.

Beide Eduktgase gelangen in den Bereich zwischen dem ersten Verdichter bzw. der ersten Verdichterstufe 12 und dem zweiten Verdichter bzw. der zweiten Verdichterstufe 26. Hier ist die Art der Verdichtung auch von dem Druckniveau der anliegenden Gase abhängig. Die Zugabe nach der ersten Verdichterstufe stellt eine von mehreren alternativen Möglichkeiten dar. Die Gase können analog zu Figur 7 der Misch- und Verdichtersektion an beliebiger Stelle zugeführt werden, in Abhängigkeit des Druckniveaus bei dem das Gas vorliegt. Der von der Destillationsvorrichtung 30 über die Rückführleitung 34 rückgeführte Recyclestrom wird hingegen in einen Bereich stromaufwärts des ersten Verdichters 12 geführt und dort zunächst verdichtet und dann ausgangsseitig des ersten Verdichters 12 mit den frisch von außen zugeführten Eduktgasen Wasserstoff und Kohlendioxid gemischt und dieses Gemisch wird in den zweiten Verdichter 26 geleitet. Das Kreisgas wird wie bereits zuvor bei der Variante von Figur 1 beschrieben vom Hochdruckabscheider 24 kommend über die Rückführleitung 25 durch den Verdichter 19 geleitet, dort verdichtet und dann in dem Bereich stromabwärts des zweiten Verdichters 26 mit dem dort erzeugten Fluidstrom gemischt und dem Methanolsynthesereaktor 22 zugeleitet.

Das Prozesswasser wird bei dieser Variante nicht recycelt, da keine Wasserstofferzeugung durch Elektrolyse im System vorgesehen ist, sondern das in der Destillationsvorrichtung 30 abgetrennte Wasser kann ähnlich wie bei der Variante gemäß Figur 1 über eine Leitung 31 vom Sumpf der Kolonne ausgehend aus dem System abgeführt werden. Gemäß einer alternativen Variante der Erfindung ist es auch möglich, einen zusätzlichen H₂-Speicher in der Anlage vorzusehen, wobei man beispielsweise das H₂ unter Druck in einem Behälter speichert. Dabei kann man beispielsweise bei einer Anlagenkonzeption wie sie zuvor unter Bezugnahme auf die Figur 3 beschrieben wurde, den H₂-Speicher im Bereich des ersten Verdichters 12 anordnen, wobei man zum kurzfristigen Ausgleich kleinerer Schwankungen stromabwärts des ersten Verdichters 12 und vor dem zweiten Verdichter 26 eine Leitung abzweigt, die zu der Eingangsseite des H₂-Speichers führt und von der Ausgangsseite des H₂-Speichers kann man eine Leitung in den Bereich vor dem ersten Verdichter 12 zurückführen. So kann zum Beispiel bei geringer Strombereitstellung die Kapazität der eigentlichen Methanol-Anlage geregelt angepasst werden. Dies ist vor allem für die Destillationskolonne 30 von Vorteil. Durch den Abzweig nach der ersten Verdichterstufe 12 wird zwar kein reiner Wasserstoff gespeichert. Der H₂-Gehalt liegt aber beispielsweise bei über 98%. Durch den Recycle-Strom gelangen weitere Komponenten wie CO₂, CO, Methanol, Dimethylether und Methylformiat hinzu. Dafür besteht aber der Vorteil, dass kein zusätzlicher Kompressor erforderlich ist.

Bei Einsatz von zum Beispiel Protonen-Austausch-Membran-Elektrolyseuren (PEM-Elektrolyseuren) fällt der H₂ bei höheren Drücken (beispielsweise von bis zu 35 bar) an und kann dann direkt ohne eine Verdichterstufe gespeichert werden.

Wenn größere Speicherkapazitäten für Wasserstoff erforderlich sind, kann man eine alternative Variante wählen. Man kann dann beispielsweise in dem in Figur 3 dargestellten Anlagenschema eine nach der Wasserelektrolyse 14 und vor dem ersten Verdichter 12 abzweigende Zweigleitung für Wasserstoff vorsehen, die zunächst zu einem weiteren Verdichter führt, um den Wasserstoff auf einen höheren Druck zu bringen und bei höherem Druck zu speichern. Stromabwärts dieses weiteren Verdichters ist dann der Wasserstoffspeicher angeordnet, von dessen Ausgang eine Leitung in den Bereich zwischen dem ersten Verdichter 12 und dem zweiten Verdichter 26 führt (siehe Anlagenschema gemäß Figur 3). Durch den zusätzlichen Verdichter kann so reiner H₂ bei höheren Drücken gespeichert werden. Die Einspeisung kann hier aber auch alternativ vor dem ersten Verdichter 12 erfolgen.

### Bezugszeichenliste

- 10: Verdampfer
- 11: Leitung für CO₂
- 12: Verdichter
- 13: Eingangsleitung für Strom
- 14: Elektrolysevorrichtung
- 15: Zuführleitung für Wasser
- 16: Wasseraufbereitung
- 17: Zuführleitung für Wasser
- 18: Leitung für Sauerstoff
- 19: Verdichter
- 20: Leitung für Wasserstoff
- 21: Leitung für Edukte
- 22: Methanolsynthesereaktor
- 23: Leitung für Produkt
- 24: Hochdruckabscheider
- 25: Rückführleitung (Kreisgasleitung)
- 26: Verdichter
- 27: Leitung
- 28: Niederdruckabscheider
- 29: Leitung
- 30: Destillationsvorrichtung
- 31: zweite Leitung
- 32: Ausgangsleitung für Methanol
- 33: Leitung
- 34: Rückführleitung
- 35: Leitung zur Nachverbrennung
- 36: Einrichtung zur katalytischen Abgasreinigung
- 37: Ausgangsleitung
- 38: Rohmethanoltank
- 39: Fackel
- 40: Abgasleitung
- 41: Abwasserleitung
- 42: Abwasserleitung
- 43: Rückführleitung für Prozesswasser
- 44: Leitung für Teilstrom
- 45: Leitung
- 46: Kühlmittelkreislauf
- 47: Abgasstrom
- 48: weitere Leitung zur Misch- und Verdichtersektion

## Patentansprüche

1. Verfahren zur Herstellung von Methanol durch Umsetzung von Kohlendioxid mit Wasserstoff, bei dem ein bei der Methanolsynthesereaktion erhaltener Produktstrom einem Hochdruckabscheider und/oder einem Niederdruckabscheider (28) zugeführt wird, in dem ein Gasstrom von einem Methanol-haltigen Produktstrom abgetrennt wird, **wobei** der Methanol-haltige Produktstrom anschließend mindestens einem Destillationsschritt (30) zugeführt wird, in dem mindestens eine Komponente, insbesondere Wasser, von dem Methanol-haltigen Produktstrom abgetrennt wird, **dadurch gekennzeichnet, dass** in dem Destillationsschritt (30) abgetrennte leichtflüchtige Komponenten teilweise oder vollständig in eine der Methanolsynthese vorgeschaltete Misch- und Verdichtersektion zurückgeführt werden.

2. Verfahren nach Anspruch 1, **dadurch gekennzeichnet, dass** ein mindestens eine abgetrennte leichtflüchtige Komponente enthaltender Gasstrom als Abgas vollständig oder nur teilweise aus dem System abgeführt wird und/oder dieser Gasstrom oder ein Teil dieses abgetrennten Gasstroms wieder in die Methanolsynthesereaktion (22) zurückgeführt wird.

3. Verfahren nach Anspruch 1 oder 2, **dadurch gekennzeichnet, dass** mindestens ein Teilstrom des von dem Methanol-haltigen Produktstrom abgetrennten Gasstroms einer katalytischen Abgasreinigung (36) zugeführt wird.

4. Verfahren nach Anspruch 3, **dadurch gekennzeichnet, dass** die katalytische Abgasreinigung (36) eine katalytische Nachverbrennung unter Zufuhr eines Zusatzbrennstoffes umfasst.

5. Verfahren nach Anspruch 4, **dadurch gekennzeichnet, dass** bei der katalytischen Nachverbrennung Sauerstoff, insbesondere durch Elektrolyse gewonnener Sauerstoff, als Zusatzbrennstoff zugeführt wird.

6. Verfahren nach einem der Ansprüche 1 bis 5, **dadurch gekennzeichnet, dass** der bei der Synthese von Methanol als Edukt eingesetzte Wasserstoff zuvor durch Elektrolyse (14) von Wasser gewonnen wurde.

7. Verfahren nach Anspruch 6, **dadurch gekennzeichnet, dass** in dem Destillationsschritt (30) oder durch Kondensation oder anderweitig von einem Methanol-haltigen Produktstrom abgetrenntes Wasser mindestens teilweise in die elektrolytische Wasserstoffgewinnung (14) aus Wasser zurückgeführt wird.

8. Verfahren nach einem der Ansprüche 1 bis 7, **dadurch gekennzeichnet, dass** das bei der Methanolsynthesereaktion (22) von flüssigem (kryogenem) Kohlendioxid ausgegangen wird.

9. Verfahren nach einem der Ansprüche 1 bis 8, **dadurch gekennzeichnet, dass** eine Verdampfung und Erwärmung des für die Methanolsynthese verwendeten Kohlendioxids vorgesehen ist, wobei hierzu die Wärme aus dem Rücklauf eines Kühlmediums genutzt wird.

10. Verfahren nach einem der Ansprüche 1 bis 9, **dadurch gekennzeichnet, dass** ein bei der Methanolsynthesereaktion (22) erhaltener Produktstrom zunächst einem Niederdruckabscheider (28) zugeführt und ein in diesem Niederdruckabscheider von dem Methanol-haltigen Produktstrom abgetrennter Gasstrom anschließend dem Destillationsschritt (30) zugeführt wird.

11. Verfahren nach einem der Ansprüche 1 bis 10, **dadurch gekennzeichnet, dass** die Misch- und Verdichtersektion eine oder mehrere hintereinandergeschaltete Verdichter oder Verdichterstufen umfasst.

12. Verfahren nach einem der Ansprüche 1 bis 11, **dadurch gekennzeichnet, dass** ein Teilstrom der in dem Destillationsschritt (30) abgetrennten leichtflüchtigen Komponenten als Abgas aus dem System abgeführt wird.

13. Verfahren nach einem der Ansprüche 1 bis 12, **dadurch gekennzeichnet, dass** ein bei der Methanolsynthesereaktion (22) erhaltener Produktstrom zunächst einem Hochdruckabscheider (24) zugeführt und ein in diesem Hochdruckabscheider von einem Methanol-haltigen Produktstrom abgetrennter Gasstrom teilweise als Kreisgas in die Methanolsynthesereaktion (22) rückgeführt und ein weiterer Teilstrom aus dem Hochdruckabscheider (24) dem Destillationsschritt (30) zugeführt wird.

14. Verfahren nach einem der Ansprüche 1 bis 13, **dadurch gekennzeichnet, dass** ein bei der Methanolsynthesereaktion (22) erhaltener Produktstrom zunächst einem Hochdruckabscheider (24) zugeführt wird, in dem ein erster Gasstrom von einem Methanol-haltigen Produktstrom abgetrennt wird und der Methanol-haltige Produktstrom danach einem Niederdruckabscheider (28) zugeführt wird, in dem ein zweiter Gasstrom von dem Methanol-haltigen Produktstrom abgetrennt wird, wobei der erste Gasstrom mindestens teilweise als Kreisgas in die Methanolsynthesereaktion (22) rückgeführt, der zweite Gasstrom anschließend dem Destillationsschritt (30) zugeführt wird und der übrige Methanol-haltige Produktstrom ebenfalls dem Destillationsschritt zugeführt wird.

15. Verfahren nach einem der Ansprüche 6 bis 14, **dadurch gekennzeichnet, dass** eine Wasseraufbereitung des für die Elektrolyse (14) als Edukt eingesetzten Wassers durch lonenaustausch und/oder Umkehrosmose vorgesehen ist.

16. Verfahren nach einem der Ansprüche 1 bis 15, **dadurch gekennzeichnet, dass** eine der Methanolsynthesereaktion (22) vorgeschaltete Misch- und Verdichtersektion vorgesehen ist, welcher von extern Kohlenmonoxid oder Wasserstoff oder ein Kohlenstoffoxide und Wasserstoff haltiges Synthesegas zugeführt wird.

17. Verfahren nach einem der Ansprüche 5 bis 16, **dadurch gekennzeichnet, dass** ein in dem Destillationsschritt (30) abgetrennter Gasstrom zu einer Misch- und Verdichtersektion zurückgeführt und kein oder nur ein minimaler Abgasstrom aus dem System abgeführt wird und in dem Destillationsschritt (30) abgetrenntes Wasser als Prozesswasser in die elektrolytische Wasserstoffgewinnung (14) zurückgeführt wird.

18. Verfahren nach einem der Ansprüche 1 bis 17, **dadurch gekennzeichnet, dass** ein in dem Destillationsschritt (30) abgetrennter Gasstrom in eine der Methanolsynthese vorgeschaltete Misch- und/oder Verdichtersektion zurückgeführt und zunächst in einem ersten Verdichter oder einer ersten Verdichterstufe verdichtet wird, nach dieser ersten Verdichtung wenigstens eines der Eduktgase, bevorzugt beide Eduktgase Kohlendioxid und Wasserstoff, mit dem verdichteten zurückgeführten Strom vereint wird, oder zunächst mit Wasserstoff vereint und dann verdichtet wird und das so entstandene vereinte Gemisch danach wenigstens einem weiteren Verdichter oder einer weiteren Verdichterstufe zugeführt und nach dieser weiteren Verdichtung der Methanolsynthesereaktion (22) zugeführt wird.

19. Verfahren nach einem der Ansprüche 1 bis 18, **dadurch gekennzeichnet, dass,** eine der Methanolsynthesereaktion (22) vorgeschaltete Misch- und Verdichtersektion vorgesehen ist, welcher von extern Kohlenmonoxid oder Wasserstoff oder ein Kohlenstoffoxide und Wasserstoff haltiges Synthesegas zugeführt wird und welcher weiterhin im System durch Elektrolyse (14) erzeugter Wasserstoff zugeführt wird und/oder welcher weiterhin aus kryogen vorliegendem Kohlendioxid nach Verdampfung erzeugtes gasförmiges Kohlendioxid zugeführt wird und/oder welcher weiterhin ein Strom in dem Destillationsschritt (30) abgetrennter und zurückgeführter leichtflüchtiger Komponenten zugeführt wird.

20. Anlage zur Herstellung von Methanol durch Umsetzung von Kohlendioxid mit Wasserstoff, umfassend wenigstens eine Reaktoreinheit (22) für die Methanolsynthese sowie umfassend wenigstens eine der Reaktoreinheit (22) nachgeschaltete erste Trennvorrichtung (24, 28) zur Abtrennung leichtflüchtiger und/oder flüssiger Bestandteile von einem Methanol-haltigen Produktstrom, **dadurch gekennzeichnet, dass** die Anlage weiterhin wenigstens eine der ersten Trennvorrichtung (24, 28) nachgeschaltete weitere Trennvorrichtung (30) zur Abtrennung leichtflüchtiger Bestandteile und Wasser durch Destillation aufweist, wobei wenigstens eine mit der Trennvorrichtung verbundene Rückführleitung (34) für eine zumindest teilweise Rückführung eines in der Trennvorrichtung (30) abgetrennten Gasstroms in einen Bereich stromaufwärts der Reaktoreinheit (22) vorgesehen ist und wenigstens eine mit der Trennvorrichtung verbundene Abgasleitung zur teilweisen oder vollständigen Abführung eines Abgasstroms aus der Anlage vorgesehen ist.

21. Anlage nach Anspruch 20, **dadurch gekennzeichnet, dass** diese weiterhin wenigstens eine Einrichtung (36) zur katalytischen Abgasreinigung eines aus der Anlage abzuführenden Abgas- und/oder Purgegasstroms umfasst, welche mit der Reaktoreinheit (22) über die Abgasleitung mittelbar oder unmittelbar in Wirkverbindung steht.

22. Anlage nach Anspruch 20 oder 21, **dadurch gekennzeichnet, dass** diese weiterhin eine Elektrolysevorrichtung (14) zur Erzeugung von Wasserstoff aus Wasser umfasst, sowie Mittel zur Zuführung des bei der Elektrolyse erzeugten Wasserstoffs zu der Reaktoreinheit (22) zur Synthese von Methanol.

23. Anlage nach Anspruch 22, **dadurch gekennzeichnet, dass** diese weiterhin wenigstens einen zwischen der Elektrolysevorrichtung (14) und der Reaktoreinheit (22) angeordneten Verdichter (12) umfasst.

24. Anlage nach einem der Ansprüche 20 bis 23, **dadurch gekennzeichnet, dass** diese als erste Trennvorrichtung wenigstens einen der Reaktoreinheit (22) nachgeschalteten Hochdruckabscheider (24) zur Abtrennung gasförmiger Bestandteile von einem Methanol-haltigen Produktstrom umfasst.

25. Anlage nach einem der Ansprüche 20 bis 24, **dadurch gekennzeichnet, dass** diese als erste Trennvorrichtung wenigstens einen der Reaktoreinheit (22) nachgeschalteten, vorzugsweise weiterhin einem Hochdruckabscheider (24) nachgeschalteten Niederdruckabscheider (28) zur Abtrennung flüssiger und/oder gasförmige Bestandteile von einem Methanol-haltigen Produktstrom umfasst.

26. Anlage nach einem der Ansprüche 20 bis 25, **dadurch gekennzeichnet, dass** die weitere Trennvorrichtung eine Destillationsvorrichtung (30) umfasst und wenigstens eine Rückführleitung (34) für die Rückführung eines in der Destillationsvorrichtung (30) abgetrennten Gasstroms in einen Bereich stromaufwärts der Reaktoreinheit (22) vorgesehen ist.

27. Anlage nach einem der Ansprüche 20 bis 26, **dadurch gekennzeichnet, dass** diese weiterhin wenigstens eine Einrichtung (16) zur Aufbereitung von Wasser durch Abtrennung von im Wasser gelösten Substanzen aufweist sowie Mittel zur Zuführung des aufbereiteten Wassers zu der Elektrolysevorrichtung (14).

28. Anlage nach einem der Ansprüche 20 bis 27, **dadurch gekennzeichnet, dass** diese wenigstens eine Einrichtung zur Bereitstellung von Kohlendioxid umfasst, wenigstens einen dieser Einrichtung nachgeschalteten Verdampfer (10) und gegebenenfalls wenigstens einen dem Verdampfer (10) nachgeschalteten Verdichter (12, 26) sowie Mittel zur Zuführung von Kohlendioxid von dem Verdampfer und/oder von dem Verdichter zu der Reaktoreinheit (22).

29. Anlage nach einem der Ansprüche 24 bis 28, **dadurch gekennzeichnet, dass** diese wenigstens eine Gasleitung von dem Hochdruckabscheider (24) zu der Einrichtung zur katalytischen Abgasreinigung (36) und/oder wenigstens eine Gasleitung von dem Niederdruckabscheider (28) zu der Einrichtung zur katalytischen Abgasreinigung (36) und/oder wenigstens eine Gasleitung (33) von der Destillationsvorrichtung (30) zu der katalytischen Abgasreinigung (36) umfasst.

30. Anlage nach einem der Ansprüche 24 bis 29, **dadurch gekennzeichnet, dass** diese wenigstens eine von dem Hochdruckabscheider (24) ausgehende Rückführleitung (25) für Gase zum Eingangsbereich der Reaktoreinheit (22) aufweist und/oder einen im Strömungsweg zwischen dem Hochdruckabscheider (24) und einem Eingangsbereich der Reaktoreinheit (22), vorzugsweise in der Rückführleitung (25), angeordneten Verdichter (26) umfasst.

31. Anlage nach Anspruch 30, **dadurch gekennzeichnet, dass** eine von der von dem Hochdruckabscheider (24) ausgehenden Rückführleitung (25) abzweigende Zweigleitung für die Zuführung mindestens eines Teilstroms der im Hochdruckabscheider (24) abgetrennten leichtflüchtigen Bestandteile in den Kopfbereich der Destillationsvorrichtung (30) vorgesehen ist.

32. Anlage nach Anspruch 31, **dadurch gekennzeichnet, dass** die Zweigleitung in eine Leitung einmündet, welche der Zuführung von im Niederdruckabscheider (28) abgetrennten leichtflüchtigen Bestandteilen zum Kopfbereich der Destillationsvorrichtung (30) dient.

33. Anlage nach einem der Ansprüche 20 bis 32, **dadurch gekennzeichnet, dass** diese eine einen oder mehrere Verdichter bzw. Verdichterstufen umfassende Misch- und Verdichtersektion umfasst, in der ein dieser von einem Verdampfer zugeführter Kohlendioxidgasstrom mit einem in der Anlage durch Wasserelektrolyse erzeugten Wasserstoffgasstrom und/oder einem von außerhalb der Anlage zugeführten Eduktgasstrom umfassend Kohlendioxid und/oder Kohlenmonoxid und/oder Wasserstoff und/oder ein Synthesegas gemischt und verdichtet wird, wobei die Misch- und Verdichtersektion der Reaktoreinheit (22) für die Methanolsynthese vorgeschaltet ist und mit dieser in Wirkverbindung steht.

34. Anlage nach einem der Ansprüche 20 bis 33, **dadurch gekennzeichnet, dass** diese einen Speicher für Wasserstoff, vorzugsweise einen Druckspeicher, umfasst, welcher stromabwärts der Wasserelektrolyse (14) angeordnet ist, wobei gegebenenfalls ein weiterer Verdichter vorgesehen ist, um dem Speicher unter erhöhtem Druck stehenden Wasserstoff zuzuführen.

## Claims

1. A process for preparing methanol by reaction of carbon dioxide with hydrogen, in which a product stream obtained in the methanol synthesis reaction is fed to a high-pressure separator and/or a low-pressure separator (28) in which a gas stream is separated off from a methanol-containing product stream, wherein the methanol-containing product stream is subsequently fed to at least one distillation step (30) in which at least one component, in particular water, is separated off from the methanol-containing product stream, **characterized in that** volatile components which have been separated off in the distillation step (30) are recirculated partly or in their entirety into a mixing and compression section located upstream of the methanol synthesis.

2. The process as claimed in claim 1, **characterized in that** a gas stream which contains at least one volatile component which has been separated off is discharged in its entirety or only partly as offgas from the system and/or this gas stream or part of this gas stream which has been separated off is recirculated to the methanol synthesis reaction (22).

3. The process as claimed in claim 1 or 2, **characterized in that** at least one substream of the gas stream which has been separated off from the methanol-containing product stream is fed to a catalytic offgas purification (36).

4. The process as claimed in claim 3, **characterized in that** the catalytic offgas purification (36) comprises a catalytic after-combustion with introduction of a supplementary combustion component.

5. The process as claimed in claim 4, **characterized in that** oxygen, in particular oxygen obtained by electrolysis, is introduced as supplementary combustion component in the catalytic after-combustion.

6. The process as claimed in any of claims 1 to 5, **characterized in that** the hydrogen used as starter material in the synthesis of methanol has been obtained beforehand by electrolysis (14) of water.

7. The process as claimed in claim 6, **characterized in that** water which has been separated off from a methanol-containing product stream in the distillation step (30) or by condensation or in another way is at least partly recirculated into the electrolytic production (14) of hydrogen from water.

8. The process as claimed in any of claims 1 to 7, **characterized in that** liquid (cryogenic) carbon dioxide is used as starter material in the methanol synthesis reaction (22).

9. The process as claimed in any of claims 1 to 8, **characterized in that** vaporization and heating of the carbon dioxide used for the methanol synthesis is provided, with the heat from the return stream of a cooling medium being used for this purpose.

10. The process as claimed in any of claims 1 to 9, **characterized in that** a product stream obtained in the methanol synthesis reaction (22) is firstly fed to a low-pressure separator (28) and a gas stream which has been separated off from the methanol-containing product stream in this low-pressure separator is subsequently fed to the distillation step (30).

11. The process as claimed in any of claims 1 to 10, **characterized in that** the mixing and compression section comprises one or more compressors or compressor stages connected in series.

12. The process as claimed in any of claims 1 to 11, **characterized in that** a substream of the volatile components separated off in the distillation step (30) is discharged as offgas from the system.

13. The process as claimed in any of claims 1 to 12, **characterized in that** a product stream obtained in the methanol synthesis reaction (22) is firstly fed to a high-pressure separator (24) and a gas stream which has been separated off from a methanol-containing product stream in this high-pressure separator is partly recirculated as recycle gas to the methanol synthesis reaction (22) and a further substream from the high-pressure separator (24) is fed to the distillation step (30).

14. The process as claimed in any of claims 1 to 13, **characterized in that** a product stream obtained in the methanol synthesis reaction (22) is firstly fed to a high-pressure separator (24) in which a first gas stream is separated off from a methanol-containing product stream and the methanol-containing product stream is then fed to a low-pressure separator (28) in which a second gas stream is separated off from the methanol-containing product stream, with the first gas stream being recirculated at least partly as recycle gas to the methanol synthesis reaction (22), the second gas stream subsequently being fed to the distillation step (30) and the remaining methanol-containing product stream likewise being fed to the distillation step.

15. The process as claimed in any of claims 6 to 14, **characterized in that** a water treatment of the water used as starter material for the electrolysis (14) by ion exchange and/or reverse osmosis is provided.

16. The process as claimed in any of claims 1 to 15, **characterized in that** a mixing and compression section is provided which is located upstream of the methanol synthesis reaction (22) and is supplied from the outside with carbon monoxide or hydrogen or a synthesis gas containing carbon oxides and hydrogen.

17. The process as claimed in any of claims 5 to 16, **characterized in that** a gas stream which has been separated off in the distillation step (30) is recirculated to a mixing and compression section and no offgas stream or only a minimal offgas stream is discharged from the system and water which has been separated off in the distillation step (30) is recirculated as process water to the electrolytic production (14) of hydrogen.

18. The process as claimed in any of claims 1 to 17, **characterized in that** a gas stream which has been separated off in the distillation step (30) is recirculated into a mixing and/or compression section located upstream of the methanol synthesis and firstly compressed in a first compressor or a first compressor stage, after this first compression at least one of the feed gases, preferably both the feed gases carbon dioxide and hydrogen, is combined with the compressed recirculated stream, or firstly combined with hydrogen and then compressed, and the resulting combined mixture is then fed to at least one further compressor or a further compressor stage and after this further compression is fed to the methanol synthesis reaction (22).

19. The process as claimed in any of claims 1 to 18, **characterized in that,** a mixing and compression section is provided which is located upstream of the methanol synthesis reaction (22) and is supplied from the outside with carbon monoxide or hydrogen or a synthesis gas containing carbon oxides and hydrogen and which is also supplied with hydrogen produced in the system by electrolysis (14) and/or is also supplied with gaseous carbon dioxide produced from cryogenic carbon dioxide by vaporization and/or is also supplied with a stream of volatile components which has been separated off in the distillation step (30) and recirculated.

20. A plant for preparing methanol by reaction of carbon dioxide with hydrogen, comprising at least one reactor unit (22) for the synthesis of methanol and comprising at least one first separation apparatus (24, 28) which is located downstream of the reactor unit (22) and has the function of separating off volatile and/or liquid constituents from a methanol-containing product stream, **characterized in that** the plant further comprises at least one further separation apparatus (30) which is located downstream of the first separation apparatus (24, 28) and has the function of separating off volatile constituents and water by distillation, with at least one return conduit (34) connected to the separation apparatus being provided for at least partial recirculation of a gas stream separated off in the separation apparatus (30) to a region upstream of the reactor unit (22) and at least one offgas conduit connected to the separation apparatus being provided for partial or complete discharge of an offgas stream from the plant.

21. The plant as claimed in claim 20, **characterized in that** it further comprises at least one apparatus (36) for catalytic offgas purification of an offgas stream and/or purge gas stream to be discharged from the plant, which apparatus is indirectly or directly in active communication with the reactor unit (22) via the offgas conduit.

22. The plant as claimed in claim 20 or 21, **characterized in that** it further comprises an electrolysis apparatus (14) for producing hydrogen from water and also means for feeding the hydrogen produced in the electrolysis to the reactor unit (22) for the synthesis of methanol.

23. The plant as claimed in claim 22, **characterized in that** it further comprises at least one compressor (12) arranged between the electrolysis apparatus (14) and the reactor unit (22).

24. The plant as claimed in any of claims 20 to 23, **characterized in that** it comprises, as first separation apparatus, at least one high-pressure separator (24) which is located downstream of the reactor unit (22) and has the function of separating gaseous constituents from a methanol-containing product stream.

25. The plant as claimed in any of claims 20 to 24, **characterized in that** this comprises, as first separation apparatus, at least one low-pressure separator (28) which is located downstream of the reactor unit (22), preferably also downstream of a high-pressure separator (24), and has the function of separating liquid and/or gaseous constituents from a methanol-containing product stream.

26. The plant as claimed in any of claims 20 to 25, **characterized in that** the further separation apparatus comprises a distillation apparatus (30) and at least one return conduit (34) for recirculating a gas stream which has been separated off in the distillation apparatus (30) to a region upstream of the reactor unit (22) is provided.

27. The plant as claimed in any of claims 20 to 26, **characterized in that** it further comprises at least one apparatus (16) for treating water by removal of substances dissolved in the water and also means for feeding the treated water to the electrolysis apparatus (14).

28. The plant as claimed in any of claims 20 to 27, **characterized in that** it comprises at least one apparatus for providing carbon dioxide, at least one vaporizer (10) located downstream of this apparatus and optionally at least one compressor (12, 26) located downstream of the vaporizer (10) and also means for feeding carbon dioxide from the vaporizer and/or from the compressor to the reactor unit (22).

29. The plant as claimed in any of claims 24 to 28, **characterized in that** it comprises at least one gas conduit from the high-pressure separator (24) to the apparatus for catalytic offgas purification (36) and/or at least one gas conduit from the low-pressure separator (28) to the apparatus for catalytic offgas purification (36) and/or at least one gas conduit (33) from the distillation apparatus (30) to the catalytic offgas purification (36).

30. The plant as claimed in any of claims 24 to 29, **characterized in that** it comprises at least one return conduit (25) which extends from the high-pressure separator (24) and conveys gases to the entry region of the reactor unit (22) and/or a compressor (26) arranged in the flow path between the high-pressure separator (24) and an entry region of the reactor unit (22), preferably in the return conduit (25).

31. The plant as claimed in claim 30, **characterized in that** a branch conduit branching off from the return conduit (25) extending from the high-pressure separator (24) is provided for feeding at least one substream of the volatile constituents separated off in the high-pressure separator (24) into the top region of the distillation apparatus (30).

32. The plant as claimed in claim 31, **characterized in that** the branch conduit opens into a conduit which serves to feed volatile constituents separated off in the low-pressure separator (28) into the top region of the distillation apparatus (30).

33. The plant as claimed in any of claims 20 to 32, **characterized in that** it comprises a mixing and compression section which comprises one or more compressors or compressor stages and in which a carbon dioxide gas stream supplied from a vaporizer is mixed with a hydrogen gas stream produced by electrolysis of water in the plant and/or a feed gas stream comprising carbon dioxide and/or carbon monoxide and/or hydrogen and/or a synthesis gas supplied from outside the plant and compressed, with the mixing and compression section being located upstream of the reactor unit (22) for the methanol synthesis and being in active communication with the latter.

34. The plant as claimed in any of claims 20 to 33, **characterized in that** it comprises a store for hydrogen, preferably a pressure store, which is arranged downstream of the electrolysis (14) of water, with a further compressor for feeding hydrogen present under elevated pressure into the store optionally being provided.

## Revendications

1. Procédé de fabrication de méthanol par mise en réaction de dioxyde de carbone avec de l'hydrogène, selon lequel un courant de produit obtenu lors de la réaction de synthèse de méthanol est amené à un séparateur à haute pression et/ou à un séparateur à basse pression (28), dans lequel un courant gazeux est séparé d'un courant de produit contenant du méthanol, **dans lequel** le courant de produit contenant du méthanol étant ensuite amené à au moins une étape de distillation (30), dans laquelle au moins un composant, notamment de l'eau, est séparé du courant de produit contenant du méthanol, **caractérisé en ce que** les composants volatils séparés dans l'étape de distillation (30) sont partiellement ou entièrement recyclés dans une section de mélange et de compression située en amont de la synthèse de méthanol.

2. Procédé selon la revendication 1, **caractérisé en ce qu'un** courant gazeux contenant au moins un composant volatil séparé est évacué entièrement ou seulement partiellement du système en tant que gaz d'échappement et/ou ce courant gazeux ou une partie de ce courant gazeux séparé est à nouveau recyclé dans la réaction de synthèse de méthanol (22).

3. Procédé selon la revendication 1 ou 2, **caractérisé en ce qu'**au moins un courant partiel du courant gazeux séparé du courant de produit contenant du méthanol est amené à une épuration catalytique du gaz d'échappement (36) .

4. Procédé selon la revendication 3, **caractérisé en ce que** l'épuration catalytique du gaz d'échappement (36) comprend une post-combustion catalytique avec apport d'un combustible supplémentaire.

5. Procédé selon la revendication 4, **caractérisé en ce que,** lors de la post-combustion catalytique, de l'oxygène, notamment de l'oxygène obtenu par électrolyse, est ajouté en tant que combustible supplémentaire.

6. Procédé selon l'une quelconque des revendications 1 à 5, **caractérisé en ce que** l'hydrogène utilisé en tant que produit de départ lors de la synthèse de méthanol a été préalablement obtenu par électrolyse (14) d'eau.

7. Procédé selon la revendication 6, **caractérisé en ce que** l'eau séparée dans l'étape de distillation (30) ou par condensation ou autrement d'un courant de produit contenant du méthanol est recyclée au moins partiellement dans la production électrolytique d'hydrogène (14) à partir d'eau.

8. Procédé selon l'une quelconque des revendications 1 a 7, **caractérisé en ce que** la réaction de synthèse de méthanol (22) part de dioxyde de carbone liquide (cryogénique).

9. Procédé selon l'une quelconque des revendications 1 à 8, **caractérisé en ce qu'**une évaporation et un chauffage du dioxyde de carbone utilisé pour la synthèse de méthanol sont prévus, la chaleur provenant du reflux d'un milieu de refroidissement étant utilisée pour cela.

10. Procédé selon l'une quelconque des revendications 1 à 9, **caractérisé en ce qu'**un courant de produit obtenu lors de la réaction de synthèse de méthanol (22) est tout d'abord amené à un séparateur à basse pression (28) et un courant gazeux séparé du courant de produit contenant du méthanol dans ce séparateur à basse pression est ensuite amené à l'étape de distillation (30).

11. Procédé selon l'une quelconque des revendications 1 à 10, **caractérisé en ce que** la section de mélange et de compression comprend un ou plusieurs compresseurs ou étages de compression montés en série.

12. Procédé selon l'une quelconque des revendications 1 à 11, **caractérisé en ce qu'**un courant partiel des composants volatils séparés dans l'étape de distillation (30) est évacué du système en tant que gaz d'échappement.

13. Procédé selon l'une quelconque des revendications 1 à 12, **caractérisé en ce qu'**un courant de produit obtenu lors de la réaction de synthèse de méthanol (22) est tout d'abord amené à un séparateur à haute pression (24) et un courant gazeux séparé d'un courant de produit contenant du méthanol dans ce séparateur à haute pression est partiellement recyclé dans la réaction de synthèse de méthanol (22) en tant que gaz circulaire et un autre courant partiel provenant du séparateur à haute pression (24) est amené à l'étape de distillation (30).

14. Procédé selon l'une quelconque des revendications 1 à 13, **caractérisé en ce qu'**un courant de produit obtenu lors de la réaction de synthèse de méthanol (22) est tout d'abord amené à un séparateur à haute pression (24), dans lequel un premier courant gazeux est séparé d'un courant de produit contenant du méthanol et le courant de produit contenant du méthanol est ensuite amené à un séparateur à basse pression (28), dans lequel un deuxième courant gazeux est séparé du courant de produit contenant du méthanol, le premier courant gazeux étant au moins partiellement recyclé en tant que gaz circulaire dans la réaction de synthèse de méthanol (22), le deuxième courant gazeux étant ensuite amené à l'étape de distillation (30) et le courant de produit restant contenant du méthanol étant également amené à l'étape de distillation.

15. Procédé selon l'une quelconque des revendications 6 à 14, **caractérisé en ce qu'**un conditionnement d'eau de l'eau utilisée en tant que produit de départ pour l'électrolyse (14) est prévu par échange d'ions et/ou osmose inverse.

16. Procédé selon l'une quelconque des revendications 1 à 15, **caractérisé en ce qu'**une section de mélange et de compression située en amont de la réaction de synthèse de méthanol (22) est prévue, à laquelle est amenée de l'extérieur du monoxyde de carbone ou de l'hydrogène ou un gaz de synthèse contenant des oxydes de carbone et de l'hydrogène.

17. Procédé selon l'une quelconque des revendications 5 à 16, **caractérisé en ce qu'**un courant gazeux séparé dans l'étape de distillation (30) est recyclé vers une section de mélange et de compression et aucun ou seulement un courant minimal de gaz d'échappement est évacué du système et l'eau séparée dans l'étape de distillation (30) est recyclée en tant qu'eau de traitement dans la production d'hydrogène électrolytique (14).

18. Procédé selon l'une quelconque des revendications 1 à 17, **caractérisé en ce qu'**un courant gazeux séparé dans l'étape de distillation (30) est recyclé dans une section de mélange et/ou de compression située en amont de la synthèse de méthanol et est tout d'abord comprimé dans un premier compresseur ou un premier étage de compression, au moins l'un des gaz de départ, de préférence les deux gaz de départ dioxyde de carbone et hydrogène, est combiné avec le courant recyclé comprimé après cette première compression, ou est tout d'abord combiné avec de l'hydrogène et ensuite comprimé, et le mélange combiné ainsi obtenu est ensuite amené au moins à un autre compresseur ou à un autre étage de compression et, après cette autre compression, est amené à la réaction de synthèse de méthanol (22).

19. Procédé selon l'une quelconque des revendications 1 à 18, **caractérisé en ce qu'**une section de mélange et de compression située en amont de la réaction de synthèse de méthanol (22) est prévue, à laquelle est amené de l'extérieur du monoxyde de carbone ou de l'hydrogène ou un gaz de synthèse contenant des oxydes de carbone et de l'hydrogène et à laquelle est en outre amené de l'hydrogène produit dans le système par électrolyse (14) et/ou à laquelle est en outre amené du dioxyde de carbone gazeux produit après évaporation à partir de dioxyde de carbone présent sous forme cryogénique et/ou à laquelle est en outre amené un courant de composants volatils séparés dans l'étape de distillation (30) et recyclés.

20. Installation pour la fabrication de méthanol par mise en réaction de dioxyde de carbone avec de l'hydrogène, comprenant au moins une unité de réacteur (22) pour la synthèse de méthanol ainsi que comprenant au moins un premier dispositif de séparation (24, 28) situé en aval de l'unité de réacteur (22) pour la séparation de constituants volatils et/ou liquides d'un courant de produit contenant du méthanol, **caractérisée en ce que** l'installation comprend en outre au moins un autre dispositif de séparation (30) situé en aval du premier dispositif de séparation (24, 28), pour la séparation de constituants volatils et d'eau par distillation, au moins une conduite de recyclage (34) reliée au dispositif de séparation étant prévue pour un recyclage au moins partiel d'un courant gazeux séparé dans le dispositif de séparation (30) dans une zone en amont de l'unité de réacteur (22) et au moins une conduite de gaz d'échappement reliée au dispositif de séparation étant prévue pour l'évacuation partielle ou totale d'un courant gazeux d'échappement hors de l'installation.

21. Installation selon la revendication 20, **caractérisée en ce que** celle-ci comprend en outre au moins un appareil (36) pour l'épuration catalytique du gaz d'échappement d'un courant de gaz d'échappement et/ou de gaz de purge à évacuer de l'installation, qui est en liaison active directe ou indirecte avec l'unité de réacteur (22) par l'intermédiaire de la conduite de gaz d'échappement.

22. Installation selon la revendication 20 ou 21, **caractérisée en ce que** celle-ci comprend en outre un dispositif d'électrolyse (14) pour la production d'hydrogène à partir d'eau, ainsi que des moyens pour amener l'hydrogène produit lors de l'électrolyse à l'unité de réacteur (22) pour la synthèse de méthanol.

23. Installation selon la revendication 22, **caractérisée en ce que** celle-ci comprend en outre au moins un compresseur (12) agencé entre le dispositif d'électrolyse (14) et l'unité de réacteur (22).

24. Installation selon l'une quelconque des revendications 20 à 23, **caractérisée en ce que** celle-ci comprend en tant que premier dispositif de séparation au moins un séparateur à haute pression (24) situé en aval de l'unité de réacteur (22) pour la séparation de constituants gazeux d'un courant de produit contenant du méthanol.

25. Installation selon l'une quelconque des revendications 20 à 24, **caractérisée en ce que** celle-ci comprend en tant que premier dispositif de séparation au moins un séparateur à basse pression (28) situé en aval de l'unité de réacteur (22), de préférence également situé en aval d'un séparateur à haute pression (24), pour la séparation de constituants liquides et/ou gazeux d'un courant de produit contenant du méthanol.

26. Installation selon l'une quelconque des revendications 20 à 25, **caractérisée en ce que** l'autre dispositif de séparation comprend un dispositif de distillation (30) et au moins une conduite de recyclage (34) est prévue pour le recyclage d'un courant gazeux séparé dans le dispositif de distillation (30) dans une zone en amont de l'unité de réacteur (22).

27. Installation selon l'une quelconque des revendications 20 à 26, **caractérisée en ce que** celle-ci comprend en outre au moins un appareil (16) pour le conditionnement d'eau par séparation de substances dissoutes dans l'eau, ainsi que des moyens pour amener l'eau conditionnée au dispositif d'électrolyse (14).

28. Installation selon l'une quelconque des revendications 20 à 27, **caractérisée en ce que** celle-ci comprend au moins un appareil pour la fourniture de dioxyde de carbone, au moins un évaporateur (10) situé en aval de cet appareil et éventuellement au moins un compresseur (12, 26) situé en aval de l'évaporateur (10), ainsi que des moyens pour amener du dioxyde de carbone depuis l'évaporateur et/ou depuis le compresseur à l'unité de réacteur (22).

29. Installation selon l'une quelconque des revendications 24 à 28, **caractérisée en ce que** celle-ci comprend au moins une conduite de gaz allant du séparateur à haute pression (24) jusqu'à l'appareil pour l'épuration catalytique du gaz d'échappement (36) et/ou au moins une conduite de gaz allant du séparateur à basse pression (28) jusqu'à l'appareil pour l'épuration catalytique du gaz d'échappement (36) et/ou au moins une conduite de gaz (33) allant du dispositif de distillation (30) jusqu'à l'appareil pour l'épuration catalytique du gaz d'échappement (36).

30. Installation selon l'une quelconque des revendications 24 à 29, **caractérisée en ce que** celle-ci comprend au moins une conduite de recyclage (25) pour des gaz, partant du séparateur à haute pression (24) jusqu'à la zone d'entrée de l'unité de réacteur (22) et/ou un compresseur (26) agencé dans le trajet d'écoulement entre le séparateur à haute pression (24) et une zone d'entrée de l'unité de réacteur (22), de préférence dans la conduite de recyclage (25).

31. Installation selon la revendication 30, **caractérisée en ce qu'**une conduite de dérivation partant en dérivation à partir de la conduite de recyclage (25) partant du séparateur à haute pression (24) est prévue pour amener au moins un courant partiel des constituants volatils séparés dans le séparateur à haute pression (24) dans la zone de tête du dispositif de distillation (30).

32. Installation selon la revendication 31, **caractérisée en ce que** la conduite de dérivation débouche dans une conduite qui sert à amener des constituants volatils séparés dans le séparateur à basse pression (28) dans la zone de tête du dispositif de distillation (30).

33. Installation selon l'une quelconque des revendications 20 à 32, **caractérisée en ce que** celle-ci comprend une section de mélange et de compression comprenant un ou plusieurs compresseurs ou étages de compression, dans laquelle un courant gazeux de dioxyde de carbone amené à celle-ci par un évaporateur est mélangé et comprimé avec un courant gazeux d'hydrogène produit dans l'installation par électrolyse d'eau et/ou un courant gazeux de départ amené de l'extérieur de l'installation comprenant du dioxyde de carbone et/ou du monoxyde de carbone et/ou de l'hydrogène et/ou un gaz de synthèse, la section de mélange et de compression étant située en amont de l'unité de réacteur (22) pour la synthèse de méthanol et étant en liaison active avec celle-ci.

34. Installation selon l'une quelconque des revendications 20 à 33, **caractérisée en ce que celle-ci** comprend un réservoir pour l'hydrogène, de préférence un réservoir sous pression, qui est agencé en aval de l'électrolyse d'eau (14), un autre compresseur étant éventuellement prévu pour amener de l'hydrogène sous pression élevée au réservoir.
